(19) Europäisches Patentamt
European Patent Office
Office européen des brevets

(11) EP 2 620 137 B1

(12) **EUROPÄISCHE PATENTSCHRIFT**

(45) Veröffentlichungstag und Bekanntmachung des Hinweises auf die Patenterteilung:
**15.11.2017 Patentblatt 2017/46**

(21) Anmeldenummer: **12153019.0**

(22) Anmeldetag: **30.01.2012**

(51) Int Cl.:
*A61K 8/37* (2006.01)
*A61Q 5/02* (2006.01)
*A61Q 11/00* (2006.01)
*A61Q 19/10* (2006.01)
*A23G 4/06* (2006.01)
*A61Q 5/00* (2006.01)
*A23G 3/36* (2006.01)
*A61K 9/68* (2006.01)
*A61K 8/49* (2006.01)
*A61Q 5/12* (2006.01)
*A61Q 17/04* (2006.01)
*A61Q 19/00* (2006.01)
*A61K 8/34* (2006.01)
*A23C 9/158* (2006.01)
*A23G 4/12* (2006.01)

(54) **Zubereitungen**

Compositions

Compositions

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**31.07.2013 Patentblatt 2013/31**

(60) Teilanmeldung:
**17166884.1 / 3 219 303**

(73) Patentinhaber: **Symrise AG**
**37603 Holzminden (DE)**

(72) Erfinder:
- **Siegel, Sven**
  **37671 Höxter (DE)**
- **Krammer, Gerhard**
  **37603 Holzminden (DE)**
- **Kindel, Günter**
  **37671 Höxter (DE)**

(74) Vertreter: **Fabry, Bernd**
**IP2 Patentanwalts GmbH**
**Schlossstrasse 523**
**41238 Mönchengladbach (DE)**

(56) Entgegenhaltungen:
**EP-A1- 0 485 170**
**EP-A1- 2 014 273**
**EP-A2- 2 168 957**
**WO-A1-2006/116436**
**US-A1- 2010 273 887**
**US-A1- 2011 081 303**

- **European Medicines Agency (EMEA): "Public statement on the use of herbal medicinal products containing pulegone and menthofuran", Scientific guideline , 23. November 2005 (2005-11-23), Seiten 1-4, XP002685143, Gefunden im Internet: URL:http://www.ema.europa.eu/docs/en_GB/document_library/Scientific_guideline/2010/04/WC500089958.pdf [gefunden am 2012-10-11]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 620 137 B1

## Beschreibung

### Gebiet der Erfindung

Die Erfindung befindet sich auf den Gebieten der Kosmetik, Pharmazie sowie der Nahrungsmittel und betrifft Zubereitungen mit kühlendem Effekt auf der Haut oder Schleimhaut, die Menthofuran zusammen mit weiteren ausgewählten Mentholverbindungen enthalten.

### Stand der Technik

Pfefferminzöl wird durch Wasserdampfdestillation von Pfefferminze (*Mentha piperita,* Lamiaceae) gewonnen. Es handelt sich um ein in der Kosmetik- und pharmazeutischen Industrie bedeutendes ätherisches Öl, das jährlich allein in Deutschland in weit über 100 Tonnen verwendet wird. Ähnlich wie das verwandte Minzöl, das aus der japanischen Minze *Menthae arvensis aetheroleum* gewonnen wird, enthält Pfefferminzöl insbesondere Menthol (35-45 %) und Menthon (15-20 %); daneben noch Menthylacetat (3-5 %), Neomenthol (2,5-3,5 %) sowie Isomenthol (3 %). Sowohl die ätherischen Öle als auch die Mentholverbindungen besitzen einen (scheinbar) kühlenden Effekt beim Auftragen auf die Haut bzw. Schleimhaut, ohne dass die Körpertemperatur beeinflusst wird; diese Wirkung ist vergleichbar mit der von Capsaicin, das umgekehrt einen scheinbar erwärmen Effekt besitzt. Menthol und einige seiner Derivate werden daher auch häufig als Analgetika oder schwache Lokalanaesthetika eingesetzt. Die Wirkung der Menthole wird im Allgemeinen darauf zurückgeführt, dass die Stoffe den Transport von Calciumionen in die Nervenzellen ermöglichen, was zu einem elektrischen Signal führt, das vom Gehirn als Kühlung wahrgenommen wird. Ein weiterer wichtiger Bestandteil der verschiedenen Minzöle stellt weiterhin das Menthofuran dar,

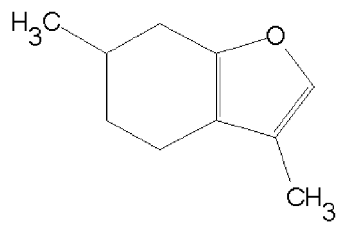

das zwar neben sehr erwünschten Noten (süß, heuartig-minzig) auch unerwünschte Geruchs-und Geschmacksnoten (Geruch: scharf und stechend; Geschmack: leicht bitter, teerartig und unangenehm) besitzt. Wegen dieser unerwünschten Noten ist es vielfach, speziell für pharmazeutische Anwendungen erforderlich, den Bestandteil beispielsweise durch Vakuumdestillation abzureichern oder ganz zu entfernen, was naturgemäß aufwendig und teuer ist.

Menthofuran besitzt zudem eine zweite Eigenschaft, die erst in Formulierungen zu Tage tritt. Es ist nämlich zu beobachten, dass Zubereitungen, die Mentholverbindungen enthalten und als Emulsionen vorliegen, in Abhängigkeit des Gehaltes an Menthofuran eine abnehmende Stabilität, speziell bei höheren Temperaturen aufweisen. Während eine handelsübliche Sonnenmilch, die Pfefferminzöl in Mengen von etwa 1 Gew.-% nicht nur als Geruchsstoff, sondern auch als "kühlende" Komponente enthält, auch bei Lagerung bei 30°C über einen Zeitraum von 24 Stunden keine Abscheidung von Öltröpfchen zeigt, wenn der Menthofurangehalt im Pfefferminzöl unter 1 Gew.-% liegt, beobachtet man bei einem Gehalt von beispielsweise 2,5 Gew.-% Menthofuran schon nach 12 Stunden erste Trübungen und nach 24 Stunden ein leichtes Aufrahmen der Rezeptur. Auch wenn dieser Effekt keinen Einfluss auf die Wirksamkeit des Sonnenschutzes hat, wird dies vom Verbraucher als Mangel des Produktes empfunden und mit einer "billigen" Rezeptur gleich gesetzt.

Die Aufgabe der vorliegenden Erfindung hat nun darin bestanden, Zubereitungen zur Verfügung zu stellen, die Menthofuran zusammen mit einem zweiten Stoff enthalten, der in der Lage ist, die unangenehmen Geruchs- und Geschmacksnoten so weit zu überdecken oder abzumildern, das nur noch die erwünschten Geruchs- und Geschmackseigenschaften des Menthofurans zum Tragen kommen, so dass eine Abtrennung dieses an sich wertvollen Aromabestandteils nicht länger erforderlich ist. Die Zusatzstoffe sollten zudem die Eigenschaft aufweisen, die negativen Eigenschaften des Menthofurans insbesondere im Zusammenhang mit der Emulsionsstabilität von Zubereitungen, die Mentholverbindungen enthalten, auszugleichen.

### Beschreibung der Erfindung

Ein erster Gegenstand der Erfindung betrifft Zubereitungen mit kühlendem Effekt, enthaltend

(a) Menthofuran und

(b) Mentholverbindungen der Formeln (II) und/oder (III)

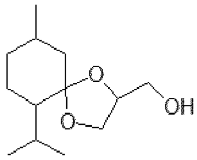

(II)

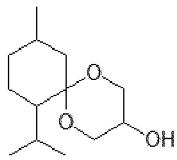

(III)

mit der Maßgabe, dass die Komponenten (a) und (b) im Gewichtsverhältnis 25:75 bis 75:25 vorhanden sind.

Überraschenderweise wurde gefunden, dass der Zusatz der oben genannten ausgewählten Mentholverbindungen nicht nur die geschmacklichen und geruchlichen Defizite des Menthofurans vollständig ausgleichen, so dass auf eine aufwendige Abtrennung nunmehr verzichtet werden kann, die Zusatzstoffe lösen auch das Problem der unzureichenden Emulsionsstabilität. Es hat sich als besonders vorteilhaft erwiesen, auf drei Teile Menthofuran ein Teil Menthone Glycerol Acetal/Ketal einzusetzen, wobei sich die Effekte schon sehr deutlich zeigen, wenn der Gehalt der Mentholverbindungen im Pfefferminzöl 1 Gew.-% übersteigt. Eine Konzentration von etwa 3 bis etwa 15 Gew.-% des Menthone Glycerol Acetals/Ketals im Pfefferminzöl ist besonders wirkungsvoll.

Bei der Untersuchung der sensorischen Eigenschaften von Mund- und Zehnpflegemitteln wurde ferner zufällig festgestellt, dass Mischungen von Menthofuran und den speziellen Mentholverbindungen gegenüber konventionellen Produkten, also etwa unbehandeltem Pfefferminzöl die Löslichkeit von Hydroxylapatit verringert und das Kristallwachstum inhibiert, so dass solche Zubereitungen auch der Demineralisierung des Zahnschmelzes entgegenwirken und die Bildung von Zahnstein verhindern.

**Mentholverbindungen**

Mentholverbindungen die im Sinne der Erfindung eingesetzt werden können, sind ausgewählt aus der Gruppe, die gebildet wird von Menthone Glyceryl Acetal (FEMA GRAS[1] 3807), Menthone Glyceryl Ketal (FEMA GRAS 3808), die die Anmelderin unter den Bezeichnungen Frescolat® MAG vertreibt sowie deren Gemischen.

[1] FEMA steht für "Flavor and Extracts Manufacturers Association" und GRAS ist definiert als "Generally Regarded As Safe". Eine FEMA GRAS Bezeichnung bedeutet, dass die so gekennzeichnete Substanz nach Standardmethode getestet und für toxikologisch unbedenklich erachtet wird.

Obschon Menthol als kühlender Stoff schon seit vielen Jahrzehnten bekannt und auch heute noch in einer Vielzahl von Anwendungen unverzichtbar ist, hat dieser Stoff doch eine ganze Reihe von Nachteilen: er ist flüchtig, hat einen strengen Geruch und bitteren Geschmack. In höheren Konzentrationen wird er nicht mehr als angenehm kühlend, sondern als stechend und brennend empfunden. Schließlich lässt sich Menthol nicht beliebig formulieren, da es mit anderen chemischen Komponenten in Wechselwirkung treten kann. Dies hat zur Entwicklung von unterschiedlichsten Mentholverbindungen geführt, von denen eine Reihe im Sinne der Erfindung in der Lage sind, die negativen Eigenschaften des Menthofurans zu neutralisieren. Alle diese Stoffe sind im Handel erhältlich und nach den üblichen Methoden der organischen Chemie erhältlich.

Das Dokument WO 2006/116436 A1 offenbart die Verwedung von Kühlstoffen mit Carboxamidstruktur, um den bitteren Geschmack von Menthol zu überdecken und den Kühleffekt bei medizinischen Kaugummis zu verstärken.

Das Dokument US 2010/0273887 A1 offenbart Zubereitungen, die eine schmerzlindernde und beruhigende Wirkung auf die Schleimhäute in Nase und Rachen ausüben und darüber hinaus weniger bitter schmecken. Dies wird durch die Kombination von Pellitorin und bestimmten kühlenden Substanzen wie zum Beispiel Menthol-Glykol-Carbonat, Menthol-Propylengylkol-Carbonat oder Menthon-Glycerin-Acetal erreicht.

Das Dokument US 2011/0081303 A1 betrifft Zahnreinigungsprodukte, die zur Verminderung des bitteren Geschmacks von Menthol spezielle Kühlwirkstoffe mit Carboxamidstruktur (WS-12) enthalten.

In einer weiteren bevorzugten Ausführungsform der Erfindung können die Zubereitungen als Komponente (c) kosmetische Zusatzstoffe enthalten, die ausgewählt sind aus der Gruppe, die gebildet wird von Tensiden, Ölkörpern,

Emulgatoren, Perlglanzwachsen, Konsistenzgebern, Verdickungsmitteln, Überfettungsmitteln, Stabilisatoren, Polymeren, Siliconverbindungen, Fetten, Wachsen, Lecithinen, Phospholipiden, UV-Lichtschutzfaktoren, Feuchthaltemitteln, biogenen Wirkstoffen, Antioxidantien, Deodorantien, Antitranspirantien, Antischuppenmitteln, Filmbildnern, Quellmitteln, Insektenrepellentien, Selbstbräunern, Tyrosininhibitoren (Depigmentierungsmittel), Hydrotropen, Solubilisatoren, Konservierungsmitteln, Parfümölen und Farbstoffen sowie deren Gemischen. Derartige Zubereitungen, insbesondere wenn sie als Emulsionen vorliegen, zeichnen sich durch eine verbesserte Lagerbeständigkeit aus.

Die erfindungsgemäßen Zubereitungen enthalten die Komponenten (a) und (b) im Gewichtsverhältnis 25:75 bis 75:25 und bevorzugt 40:60 bis 60:40 Die Komponenten (a+b) und (c) können im Gewichtsverhältnis 0.01:99,9 bis 2:98, vorzugsweise 0,5:99,5 bis 1,5: 98,5 und insbesondere ungefähr 1:99 enthalten sein.

**Gewerbliche Anwendbarkeit**

**Kosmetische und/oder pharmazeutische Zubereitungen**

Ein weiterer Gegenstand der vorliegenden Erfindung betrifft kosmetische Zubereitungen, enthaltend

(a) Menthofuran,

(b) Mentholverbindungen der Formeln (II) und/oder (III) sowie

(c) einen für kosmetische Anwendungen zugelassenen Träger,

mit der Maßgabe, dass die Komponenten (a) und (b) im Gewichtsverhältnis 25:75 bis 75:25 vorhanden sind.

Bei den kosmetischen Produkten handelt es sich vorzugsweise um Hautpflegemittel, Haarpflegemittel, Körperpflegemittel, Sonnenschutzmittel sowie Mund- und Zahnpflegemittel. Besonders bevorzugt sind solche Zubereitungen, die als Emulsionen, Mikroemulsionen oder PIT-Emulsionen vorliegen.

Ein weiterer Gegenstand der vorliegenden Erfindung betrifft pharmazeutische Zubereitungen, enthaltend

(a) Menthofuran,

(b) Mentholverbindungen der Formeln (I), (II) und/oder (III) sowie

(c) einen für pharmazeutische Anwendungen zugelassenen Träger

zur Behandlung von Erkältungszuständen, mit der Maßgabe, dass die Komponenten (a) und (b) im Gewichtsverhältnis 25:75 bis 75:25 vorhanden sind.

Bei den pharmazeutischen Produkten handelt es sich vorzugsweise um Lutschpastillen, Erkältungsbonbons, Erkältungssäfte, Erkältungssalben und Erkältungssprays.

Die kosmetischen bzw. pharmazeutischen Träger können dabei vorzugsweise ausgewählt sein aus der Gruppe, die gebildet wird von Wasser, Alkoholen mit 2 bis 6 Kohlenstoffatomen, Polyolen mit 1 bis 10 Kohlenstoffatomen und 2 bis 4 Hydroxylgruppen sowie Ölkörpern. Besonders bevorzugt sind neben Wasser, Ethanol, Isopropylalkohol, Ethylenglykol, Propylenglykol, Glycerin, Trimethylolpropan, Pentaerythritol sowie Ester von linearen oder verzweigten, gesättigten und insbesondere ungesättigten Fettsäuren mit 6 bis 22 und vorzugsweise 8 bis 18 Kohlenstoffatomen mit Alkoholen mit 1 bis 6 Kohlenstoffatomen.

Die erfindungsgemäßen kosmetischen und/oder pharmazeutischen Zubereitungen enthalten die Komponenten (a) und (b) im Gewichtsverhältnis 25:75 bis 75:25 und bevorzugt 40:60 bis 60:40 Die Komponenten (a+b) und (c) können im Gewichtsverhältnis 0.01:99,9 bis 2:98, vorzugsweise 0,5:99,5 bis 1,5: 98,5 und insbesondere ungefähr 1:99 enthalten sein.

Die erfindungsgemäßen kosmetischen und/oder pharmazeutischen Mittel können weitere typische Hilfs- und Zusatzstoffe enthalten, wie beispielsweise milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, UV-Lichtschutzfaktoren, Feuchthaltemittel, biogene Wirkstoffe, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten.

***Tenside***

Als oberflächenaktive Stoffe können anionische, nichtionische, kationische und/oder amphotere bzw. zwitterionische Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Alkylethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

***Ölkörper***

Als Ölkörper kommen beispielsweise Guerbetalkohole auf Basis von Fettalkoholen mit 6 bis 18, vorzugsweise 8 bis 10 Kohlenstoffatomen, Ester von linearen $C_6$-$C_{22}$-Fettsäuren mit linearen oder verzweigten $C_6$-$C_{22}$-Fettalkoholen bzw. Ester von verzweigten $C_6$-$C_{13}$-Carbonsäuren mit linearen oder verzweigten $C_6$-$C_{22}$-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen $C_6$-$C_{22}$-Fettsäuren mit verzweigten Alkoholen, insbesondere 2-Ethylhexanol, Ester von $C_{18}$-$C_{38}$-Alkylhydroxycarbonsäuren mit linearen oder verzweigten $C_6$-$C_{22}$-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis $C_6$-$C_{10}$-Fettsäuren, flüssige Mono-/Di-/Triglyceridmischungen auf Basis von $C_6$-$C_{18}$-Fettsäuren, Ester von $C_6$-$C_{22}$-Fettalkoholen und/oder Guerbetalkoholen mit aromatischen Carbonsäuren, insbesondere Benzoesäure, Ester von $C_2$-$C_{12}$-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte $C_6$-$C_{22}$-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten $C_6$-$C_{22}$-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

***Emulgatoren***

Als Emulgatoren kommen beispielsweise nichtionogene Tenside aus mindestens einer der folgenden Gruppen in Frage:

- Anlagerungsprodukte von 2 bis 30 Mol Ethylenoxid und/ oder 0 bis 5 Mol Propylenoxid an lineare Fettalkohole mit

8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;

- Alkyl- und/oder Alkenyloligoglykoside mit 8 bis 22 Kohlenstoffatomen im Alk(en)ylrest und deren ethoxylierte Analoga;
- Anlagerungsprodukte von 1 bis 15 Mol Ethylenoxid an Ricinusöl und/oder gehärtetes Ricinusöl;
- Anlagerungsprodukte von 15 bis 60 Mol Ethylenoxid an Ricinusöl und/oder gehärtetes Ricinusöl;
- Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
- Mono-, Di- und Trialkylphosphate sowie Mono-, Di- und/oder Tri-PEG-alkylphosphate und deren Salze;
- Wollwachsalkohole;
- Polysiloxan-Polyalkyl-Polyether-Copolymere bzw. entsprechende Derivate;
- Block-Copolymere z.B. Polyethylenglycol-30 Dipolyhydroxystearate;
- Polymeremulgatoren, z.B. Pemulen-Typen (TR-1,TR-2) von Goodrich oder Cosmedia® SP von Cognis;
- Polyalkylenglycole sowie
- Glycerincarbonat.

Im Folgenden werden besonders geeignete Emulgatoren näher erläutert:

**(a) Alkoxylate**
Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. $C_{12/18}$-Fettsäuremono- und -diester von Anlagerungsprodukten von Ethylenoxid an Glycerin sind als Rückfettungsmittel für kosmetische Zubereitungen bekannt.

**(b) Alkyl- und/oder Alkenyloligoglykoside**
Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

**(c) Partialglyceride**
Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

**(d) Sorbitanester**

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesqui-tartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitan-dimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

**(e) Polyglycerinester**

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

**(f) Anionische Emulgatoren**

Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

**(g) Amphotere und kationische Emulgatoren**

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethyl-ammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung Co*camidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer $C_{8/18}$-Alkyl- oder Acylgruppe im Molekül mindestens eine freie Aminogruppe und mindestens eine -COOH- oder -$SO_3$H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das $C_{12/18}$-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

***Fette und Wachse***

Typische Beispiele für Fette sind Glyceride, d.h. feste oder flüssige pflanzliche oder tierische Produkte, die im Wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

***Perlglanzwachse***

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

***Konsistenzgeber und Verdickungsmittel***

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und - diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone® Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

***Überfettungsmittel***

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

***Stabilisatoren***

Als Stabilisatoren können Metallsalze von Fettsäuren, wie z.B. Magnesium-, Aluminium- und/oder Zinkstearat bzw. -ricinoleat eingesetzt werden.

***Polymere***

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

***Siliconverbindungen***

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

***UV-Lichtschutzfaktoren***

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. Üblicherweise sind die UV-Lichtschutzfaktoren in Mengen von 0,1 bis 5 und vorzugsweise 0,2 bis 1 Gew.-% zugegen. UVB-Filter können öllöslich oder wasserlöslich sein. Als öllösliche Substanzen sind z.B. zu nennen:

- 3-Benzylidencampher bzw. 3-Benzylidennorcampher und dessen Derivate, z.B. 3-(4-Methylbenzyliden)campher beschrieben;
- 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexyl-ester;
- Triazinderivate, wie z.B. 2,4,6-Trianilino-(p-carbo-2'-ethyl-1'-hexyloxy)-1,3,5-triazin und Octyl Triazon oder Dioctyl Butamido Triazone (Uvasorb® HEB);
- Propan-1,3-dione, wie z.B. 1-(4-tert.Butylphenyl)-3-(4'methoxyphenyl)propan-1,3-dion;
- Ketotricyclo(5.2.1.0)decan-Derivate.

Als wasserlösliche Substanzen kommen in Frage:

- 2-Phenylbenzimidazol-5-sulfonsäure und deren Alkali-, Erdalkali-, Ammonium-, Alkylammonium-, Alkanolammonium- und Glucammoniumsalze;
- 1H-Benzimidazole-4,6-Disulfonic Acid, 2,2'-(1,4-Phenylene)Bis-, Disodium Salt (Neo Heliopan® AP)
- Sulfonsäurederivate von Benzophenonen, vorzugsweise 2-Hydroxy-4-methoxybenzophenon-5-sulfonsäure und ihre Salze;

- Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 2-(4-Diethylamino-2-hydroxybenzoyl)-benzoic acid hexylester (Uvinul® A Plus), 1-Phenyl-3-(4'-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans,, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000, Eusolex® T, Eusolex® T-ECO, Eusolex® T-S, Eusolex® T-Aqua, Eusolex® T-45D (alle Merck), Uvinul $TiO_2$ (BASF). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid wie z.B. Z-COTE® oder Z-COTE HP1® verwendet.

### *Feuchthaltemittel*

Feuchthaltemittel dienen zur weiteren Optimierung der sensorischen Eigenschaften der Zusammensetzung sowie zur Feuchtigkeitsregulierung der Haut. Gleichzeitig wird die Kältestabilität der erfindungsgemäßen Zubereitungen, insbesondere im Falle von Emulsionen, erhöht. Die Feuchthaltemittel sind üblicherweise in einer Menge von 0,1 bis 15 Gew.-%, vorzugsweise 1 bis 10 Gew.-%, und insbesondere 5 bis 10 Gew.-% enthalten.

Erfindungsgemäß geeignet sind u.a. Aminosäuren, Pyrrolidoncarbonsäure, Milchsäure und deren Salze, Lactitol, Harnstoff und Harnstoffderivate, Harnsäure, Glucosamin, Kreatinin, Spaltprodukte des Kollagens, Chitosan oder Chitosansalze/-derivate, und insbesondere Polyole und Polyolderivate (z. B. Glycerin, Diglycerin, Triglycerin, Ethylenglycol, Propylenglycol, Butylenglycol, Erythrit, 1,2,6-Hexantriol, Polyethylenglycole wie PEG-4, PEG-6, PEG-7, PEG-8, PEG-9, PEG-10, PEG-12, PEG-14, PEG-16, PEG-18, PEG-20), Zucker und Zuckerderivate (u.a. Fructose, Glucose, Maltose, Maltitol, Mannit, Inosit, Sorbit, Sorbitylsilandiol, Sucrose, Trehalose, Xylose, Xylit, Glucuronsäure und deren Salze), ethoxyliertes Sorbit (Sorbeth-6, Sorbeth-20, Sorbeth-30, Sorbeth-40), Honig und gehärteter Honig, gehärtete Stärkehydrolysate sowie Mischungen aus gehärtetem Weizenprotein und PEG-20-Acetatcopolymer. Erfindungsgemäß bevorzugt geeignet als Feuchthaltemittel sind Glycerin, Diglycerin, Triglycerin und Butylenglycol.

### *Biogene Wirkstoffe und Antioxidantien*

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt und Vitaminkomplexe zu verstehen.

Antioxidantien unterbrechen die photochemische Reaktionskette, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide,

Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. VitaminE-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, $ZnSO_4$) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

***Deodorantien und keimhemmende Mittel***

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

**(a) Keimhemmende Mittel**

Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4-dichlorphenyl)harnstoff, 2,4,4'-Trichlor-2'-hydroxy-diphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-Iod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

**(b) Enzyminhibitoren**

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropy-Icitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

**(c) Geruchsabsorber**

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, $\alpha$-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, $\beta$-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

**(d) Antitranspirantien**

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:

- adstringierende Wirkstoffe,
- Ölkomponenten,
- nichtionische Emulgatoren,
- Coemulgatoren,
- Konsistenzgeber,
- Hilfsstoffe wie z. B. Verdicker oder Komplexierungsmittel und/oder
- nichtwässrige Lösungsmittel wie z. B. Ethanol, Propylenglykol und/oder Glycerin. Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichloro-hydrat, Aluminium-Zirkonium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
- entzündungshemmende, hautschützende oder wohlriechende ätherische Öle,
- synthetische hautschützende Wirkstoffe und/oder
- öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

***Filmbildner***

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

***Antischuppenwirkstoffe***

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

***Quellmittel***

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in Cosm.Toil. 108, 95 (1993) entnommen werden.

***Insektenrepellentien***

Als Insekten-Repellentien kommen N,N-Diethyl-m-toluamid, 1,2-Pentandiol oder Ethyl Butylacetylaminopropionate in Frage. Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

***Inhaltsstoffe für Mund- und Zahnpflegemittel***

Unter Zahnpasten oder Zahncremes werden im allgemeinen gelförmige oder pastöse Zubereitungen aus Wasser, Verdickungsmitteln, Feuchthaltemitteln, Schleif- oder Putzkörpern, Tensiden, Süßmitteln, Aromastoffen, deodorierenden Wirkstoffen sowie Wirkstoffen gegen Mund- und Zahnerkrankungen verstanden. In die erfindungsgemäßen Zahnpasten können alle üblichen Putzkörper, wie z. B. Kreide, Dicalciumphosphat, unlösliches Natriummetaphosphat, Aluminiumsilikate, Calciumpyrophosphat, feinteilige Kunstharze, Kieselsäuren, Aluminiumoxid und Aluminiumoxidtrihydrat eingesetzt werden.

Bevorzugt geeignete Putzkörper für die erfindungsgemäßen Zahnpasten sind vor allem feinteilige Xerogelkieselsäuren, Hydrogelkieselsäuren, Fällungskieselsäuren, Aluminiumoxidtrihydrat und feinteiliges alpha -Aluminiumoxid oder Mischungen dieser Putzkörper in Mengen von 15 bis 40 Gew.-% der Zahnpasta. Als Feuchthaltemittel kommen vorwiegend niedermolekulare Polyethylenglykole, Glycerin, Sorbit oder Mischungen dieser Produkte in Mengen bis zu 50 Gew.-% in Frage. Unter den bekannten Verdickungsmitteln sind die verdickenden, feinteiligen Gelkieselsäuren und Hydrokolloide, wie z. B. Carboxymethylcellulose, Hydroxyethylcellulose, Hydroxypropylguar, Hydroxyethylstärke, Polyvinylpyrrolidon, hochmolekulares Polyethylenglykol, Pflanzengummen wie Traganth, Agar-Agar, Carragheenmoos, Gummiarabicum, Xantham-Gum und Carboxyvinylpolymere (z. B. Carbopol®-Typen) geeignet. Zusätzlich zu den Mischungen aus menthofuran und Mentholverbindungen können die Mund- und Zahnpflegemittel insbesondere oberflächenaktive Stoffe, bevorzugt anionische und nichtionische schaumstarke Tenside, wie die bereits oben genannten Stoffe, insbesondere aber Alkylethersulfat-Salze, Alkylpolyglucoside und deren Gemische.

Weitere übliche Zahnpastenzusätze sind:

- Konservierungsmittel und antimikrobielle Stoffe wie z. B. p- Hydroxybenzösäuremethyl-, -ethyl- oder -propylester, Natriumsorbat, Natriumbenzoat, Bromchlorophen, Phenylsalicylsäureester, Thymol und dergleichen;
- Antizahnsteinwirkstoffe, z. B. Organophosphate wie 1-Hydroxyethan- 1.1-diphosphonsäure, 1-Phosphonpropan-1,2,3-tricarbonsäure und andere, die z. B. aus US 3,488,419, DE 2224430 A1 und DE 2343196 A1 bekannt sind;
- andere karieshemmende Stoffe wie z. B. Natriumfluorid, Natriummonofluorphosphat, Zinnfluorid;
- Süssungsmittel, wie z. B. Saccharin-Natrium, Natrium-Cyclamat, Sucrose, Lactose, Maltose, Fructose oder Apartam®, (L-Aspartyl- L-phenylalanin-methylester), Stivia-extrakte oder deren süßenden Bestandteile, insbesondere Ribeaudioside;
- Zusätzliche Aromen wie z. B. Eukalyptusöl, Anisöl, Fenchelöl, Kümmelöl, Methylacetat, Zimtaldehyd, Anethol, Vanillin, Thymol sowie Mischungen dieser und anderer natürlicher und synthetischer Aromen;
- Pigmente wie z. B. Titandioxid;
- Farbstoffe;

- Puffersubstanzen wie z. B. primäre, sekundäre oder tertiäre Alkaliphosphate oder Citronensäure/Natriumcitrat;
- wundheilende und entzündungshemmende Stoffe wie z. B. Allantoin, Harnstoff, Azulen, Kamillenwirkstoffe und Acetylsalicylsäurederivate.

Eine bevorzugte Ausführung der kosmetischen Zubereitungen sind Zahnpasten in Form einer wässrigen, pastösen Dispersion, enthaltend Poliermittel, Feuchthaltemittel, Viskositätsregulatoren und gegebenenfalls weitere übliche Komponenten, sowie die Mischung aus Menthofuran und Mentholverbindungen in Mengen von 0,5 bis 2 Gew.-% enthalten.

In Mundwässern ist eine Kombination mit wässrig-alkoholischen Lösungen verschiedener Grädigkeit von ätherischen Ölen, Emulgatoren, adstringierenden und tonisierenden Drogenauszügen, zahnsteinhemmenden, antibakteriellen Zusätzen und Geschmackskorrigentien ohne weiteres möglich. Eine weitere bevorzugte Ausführung der Erfindung ist ein Mundwasser in Form einer wässrigen oder wässrig-alkoholischen Lösung enthaltend die Mischung aus Menthofuran und Mentholverbindungen in Mengen von 0,5 bis 2 Gew.-%. In Mundwässern, die vor der Anwendung verdünnt werden, können mit, entsprechend dem vorgesehenen Verdünnungsverhältnis, höheren Konzentrationen ausreichende Effekte erzielt werden.

***Hydrotrope***

Zur Verbesserung des Fließverhaltens können ferner Hydrotrope, wie beispielsweise Ethanol, Isopropylalkohol, oder Polyole eingesetzt werden; diese Stoffe entsprechen weitgehend den eingangs geschildern Trägern. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind

- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

***Konservierungsmittel***

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

***Parfümöle und Aromen***

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen),

Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

***Farbstoffe***

Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S.81-106 zusammengestellt sind. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

**Nahrungsmittelzubereitungen**

Ein weiterer Gegenstand der Erfindung betrifft Nahrungsmittelzubereitungen, enthaltend

(a) Menthofuran,

(b) Mentholverbindungen der Formeln (I), (II) und/oder (III) sowie

(c) einen für Nahrungsmittelzwecke zugelassenen Träger,

mit der Maßgabe, dass die Komponenten (a) und (b) im Gewichtsverhältnis 25:75 bis 75:25 vorhanden sind.

Die Träger können dabei ausgewählt sein aus der Gruppe, die gebildet wird von Wasser, Ethanol und Glycerin.

Bei den Nahrungsmittelzubereitungen handelt es sich vorzugsweise um Getränke, Milchprodukten, Backwaren, sowie insbesondere Kaugummis und Bonbons.

Die erfindungsgemäßen Zubereitungen können die Komponenten (a) und (b) im Gewichtsverhältnis 25:75 bis 75:25 und besonders bevorzugt 40:60 bis 60:40 enthalten. Die Komponenten (a+b) und (c) können im Gewichtsverhältnis 0.01:99,9 bis 2:98, vorzugsweise 0,5:99,5 bis 1,5: 98,5 und insbesondere ungefähr 1:99 enthalten sein.

**Kaugummis**

Bei den bevorzugten Nahrungsmittelzubereitungen, die als Geschmacksstoffe die Mischungen aus Menthofuran und den Mentholverbindungen enthalten, handelt es sich um Kaugummis. Diese Produkte enthalten typischerweise eine wasserunlösliche und eine wasserlösliche Komponente.

### *Wasserunlösliche Basis*

Die wasserunlösliche Basis, die auch als "Gummibasis" bezeichnet wird. umfasst üblicherweise natürliche oder synthetische Elastomere, Harze, Fette und Öle, Weichmacher, Füllstoffe, Farbstoffe sowie gegebenenfalls Wachse. Der Anteil der Basis an der Gesamtzusammensetzung macht üblicherweise 5 bis 95, vorzugsweise 10 bis 50 und insbesondere 20 bis 35 Gew.-% aus. In einer typischen Ausgestaltungsform der Erfindung setzt sich die Basis aus 20 bis 60 Gew.-% synthetischen Elastomeren, 0 bis 30 Gew.-% natürlichen Elastomeren, 5 bis 55 Gew.-% Weichmachern, 4 bis 35 Gew.-% Füllstoffe und in untergeordneten Mengen Zusatzstoffe wie Farbstoffe, Antioxidantien und dergleichen zusammen, mit der Maßgabe, dass sie allenfalls in geringen Mengen wasserlöslich sind.

Als geeignete synthetische Elastomere kommen beispielsweise Polyisobutylene mit durchschnittlichen Molekulargewichten (nach GPC) von 10.000 bis 100.000 und vorzugsweise 50.000 bis 80.000, Isobutylen-Isopren-Copolymere ("Butyl Elastomere"), Styrol-Butadien-Copolymere (Styrol:Butadien-Verhältnis z.B. 1: 3 bis 3: 1), Polyvinylacetate mit durchschnittlichen Molekulargewichten (nach GPC) von 2.000 bis 90.000 und vorzugsweise 10.000 bis 65.000, Polyisoprene, Polyethylen, Vinylacetat-Vinyllaurat-Copolymere und deren Gemische. Beispiele für geeignete natürliche Elastomere sind Kautschuks wie etwa geräucherter oder flüssiger Latex oder Guayule sowie natürliche Gummistoffe wie Jelutong, Lechi caspi, Perillo, Sorva, Massaranduba balata, Massaranduba chocolate, Nispero, Rosindinba, Chicle, Gutta hang 1kang sowie deren Gemische. Die Auswahl der synthetischen und natürlichen Elastomere und deren Mischungsverhältnisse richtet sich im Wesentlichen danach, ob mit den Kaugummis Blasen erzeugt werden sollen ("bubble gums") oder nicht. Vorzugsweise werden Elastomergemische eingesetzt, die Jelutong, Chicle, Sorva und Massaranduba enthalten.

In den meisten Fällen erweisen sich die Elastomere in der Verarbeitung als zu hart oder zu wenig verformbar, so dass es sich als vorteilhaft erwiesen hat, spezielle Weichmacher mitzuverwenden, die natürlich insbesondere auch alle Anforderungen an die Zulassung als Nahrungsmittelzusatzstoffe erfüllen müssen. In dieser Hinsicht kommen vor allem Ester von Harzsäuren in Betracht, beispielsweise Ester von niederen aliphatischen Alkoholen oder Polyolen mit ganz oder teilweise gehärteten, monomeren oder oligomeren Harzsäuren. Insbesondere werden für diesen Zweck die Methyl-, Glycerin-, oder Pentareythritester sowie deren Gemische eingesetzt. Alternativ kommen auch Terpenharze in Betracht, die sich von alpha-Pinen, beta-Pinen, delta-Limonen oder deren Gemischen ableiten können.

Als Füllstoffe oder Texturiermittel kommen Magnesium- oder Calciumcarbonat, gemahlener Bimsstein, Silicate, speziell Magnesium- oder Aluminiumsilicate, Tone, Aluminiumoxide. Talkum, Titandioxid, Mono-, Di- und Tricalciumphosphat sowie Cellulosepolymere.

Geeignete Emulgatoren sind Talg, gehärteter Talg, gehärtete oder teilweise gehärtete pflanzliche Öle, Kakaobutter, Partialglyceride, Lecithin, Triacetin und gesättigte oder ungesättigte Fettsäuren mit 6 bis 22 und vorzugsweise 12 bis 18 Kohlenstoffatomen sowie deren Gemische.

Als Farbstoffe und Weißungsmittel kommen beispielsweise die für die Färbung von Lebensmitteln zugelassenen FD und C-Typen, Pflanzen- und Fruchtextrakte sowie Titandioxid in Frage.

Die Basismassen können Wachse enthalten oder wachsfrei sein; Beispiele für wachsfreie Zusammensetzungen finden sich unter anderem in der Patentschrift US 5,286,500, auf deren Inhalt hiermit ausdrücklich Bezug genommen wird.

### *Wasserlösliche Bestandteile*

Zusätzlich zu der wasserunlöslichen Gummibasis enthalten Kaugummizubereitungen regelmäßig einen wasserlösliche Anteil, der beispielsweise von Softener, Süßstoffen, Füllstoffen, Geschmacksstoffen, Geschmacksverstärkern, Emulgatoren, Farbstoffen, Säuerungsmitteln, Antioxidantien und dergleichen gebildet werden, hier mit der Maßgabe, dass die Bestandteile eine wenigstens hinreichende Wasserlöslichkeit besitzen. In Abhängigkeit der Wasserlöslichkeit der speziellen Vertreter können demnach einzelne Bestandteile sowohl der wasserunlöslichen wie auch der wasserlöslichen Phase angehören. Es ist jedoch auch möglich, Kombinationen beispielsweise eines wasserlöslichen und eines wasserunlöslichen Emulgators einzusetzen, wobei sich die einzelnen Vertreter, dann in unterschiedlichen Phasen befinden. Üblicherweise macht der wasserunlösliche Anteil 5 bis 95 und vorzugsweise 20 bis 80 Gew.-% der Zubereitung aus.

Wasserlösliche Softener oder Plastifiziermittel werden den Kaugummizusammensetzungen hinzugegeben um die Kaubarkeit und das Kaugefühl zu verbessern und sind in den Mischungen typischerweise in Mengen von 0,5 bis 15 Gew.-% zugegen. Typische Beispiele sind Glycerin, Lecithin sowie wässrige Lösungen von Sorbitol, gehärteten Stärkehydrolysaten oder Kornsirup.

Als Süßstoffe kommen sowohl zuckerhaltige wie zuckerfreie Verbindungen in Frage, die in Mengen von 5 bis 95, vorzugsweise 20 bis 80 und insbesondere 30 bis 60 Gew.-% bezogen auf die Kaugummizusammensetzung eingesetzt werden. Typische Saccharid-Süssstoffe sind Sucrose, Dextrose, Maltose, Dextrin, getrockneter Invertzucker, Fructose, Levulose, Galactose, Kornsirup sowie deren Gemische. Als Zuckerersatzstoffe kommen Sorbitol, Mannitol, Xylitol, gehärtete Stärkehydrolysate, Maltitol und deren Gemische in Frage. Weiterhin kommen als Zusatzstoffe auch sogenannte HIAS ("High Intensity Articifical Sweeteners") in Betracht, wie beispielsweise Sucralose, Aspartam, Acesulfamsalze, Alitam, Saccharin und Saccharinsalze, Cyclamsäure und deren Salze, Glycyrrhizine, Dihydrochalcone, Thaumatin, Monellin und dergleichen alleine oder in Abmischungen. Besonders wirksam sind auch die hydrophoben HIAS, die Gegenstand der internationalen Patentanmeldung WO 2002 091849 A1 (Wrigleys) sowie Stevia Extrakte und deren aktiven Bestandteile, insbesondere Ribeaudiosid A sind. Die Einsatzmenge dieser Stoffe hängt in erster Linie von ihrem Leistungsvermögen ab und liegt typischerweise im Bereich von 0,02 bis 8 Gew.-%.

Insbesondere für die Herstellung kalorienarmer Kaugummis eignen sich Füllstoffe wie beispielsweise Polydextrose, Raftilose, Rafitilin, Fructooligosaccharide (NutraFlora), Palatinoseoligosaaccharide, Guar Gum Hydrolysate (Sun Fiber) sowie Dextrine.

Die Auswahl an weiteren Geschmacksstoffen ist praktisch unbegrenzt und für das Wesen der Erfindung unkritisch. Üblicherweise liegt der Gesamtanteil aller Geschmacksstoffe bei 0,1 bis 15 und vorzugsweise 0,2 bis 5 gew.-% bezogen auf die Kaugummizusammensetzung. Geeignete weitere Geschmacksstoffe stellen beispielsweise essentielle Öle, synthetische Aromen und dergleichen dar, wie etwa Anisöl, Sternanisöl, Kümmelöl, Eukalyptusöl, Fenchelöl, Citronenöl, Wintergrünöl, Nelkenöl, und dergleichen, wie sie auch beispielsweise in Mund- und Zahnpflegemittel Verwendung finden.

Die Kaugummis können des weiteren Hilfs- und Zusatzstoffe enthalten, die beispielsweise für die Zahnpflege, speziell zur Bekämpfung von Plaqü und Gingivitis geeignet sind, wie z.B. Chlorhexidin, CPC oder Trichlosan. Weiter können pH-Regulatoren (z.B. Puffer oder Harnstoff), Wirkstoffe gegen Karies (z.B. Phosphate oder Fluoride), biogene Wirkstoffe (Antikörper, Enzyme, Koffein, Pflanzenextrakte) enthalten sein, solange diese Stoffe für Nahrungsmittel zugelassen sind und nicht in unerwünschter Weise miteinander in Wechselwirkung treten.

**Kapseln**

Die Zubereitungen aus Menthofuran und den Mentholverbindungen alleine oder aber die fertig konfektionierten kosmetischen, pharmazeutischen und Nahrungsmittelzubereitungen können auch in verkapselter Form vorliegen. Neben üblichen Makrokapseln auf Basis von Gelatine kommen dabei vor allem auch so genannte Mikro- oder Nanokapseln in Betracht. Darunter werden vom Fachmann sphärische Aggregate mit einem Durchmesser im Bereich von etwa 0,0001 bis etwa 5 und vorzugsweise 0,005 bis 0,5 mm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden geschmolzene Wachse in einer Matrix aufgenommen ("microsponge"), die als Mikropartikel zusätzlich mit filmbildenden Polymeren umhüllt sein können. Nach einem dritten Verfahren werden Partikel abwechselnd mit Polyelektrolyten unterschiedlicher Ladung beschichtet ("layer-by-layer"-Verfahren). Die mikroskopisch kleinen Kapseln lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccharide, wie Stärke oder Dextran, Polypeptide, Proteinhydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon. Beispiele für Mikrokapseln des Stands der Technik sind folgende Handelsprodukte (in Klammern angegeben ist jeweils das Hüllmaterial) : *Hallcrest Microcapsules* (Gelatine, Gummi Arabicum), *Coletica Thalaspheres* (maritimes Collagen), *Lipotec Millicapseln* (Alginsäure, Agar-Agar), *Induchem Unispheres* (Lactose, mikrokristalline Cellulose, Hydroxypropy-Imethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar) und *Kuhs Probiol Nanospheres* (Phospholipide) sowie *Primaspheres* und *Primasponges* (Chitosan, Alginate) und *Primasys* (Phospholipide). Besonders interessant für die Verkapselung von Zubereitungen für kosmetische Anwendungen sind Koazervate von kationischen Polymeren, insbesondere von Chitosan, mit anioniscchen polymeren, speziell Alginaten. Entsprechende Verfahren sind beispielsweise in den Druckschriften WO 2001 001926**,** WO 2001 001927**,** WO 2001 001928 **und** WO 2001 001929 (Cognis) beschrieben.

***Gelbildner***

Mikrokapseln enthalten die Wirkstoffe häufig in einer Gelphase gelöst oder dispergiert. Als Gelbildner werden vorzugsweise solche Stoffe in Betracht gezogen, welche die Eigenschaft zeigen in wässriger Lösung bei Temperaturen oberhalb von 40 °C Gele zu bilden. Typische Beispiele hierfür sind Heteropolysaccharide und Proteine. Als thermogelierende Heteropolysaccharide kommen vorzugsweise Agarosen in Frage, welche in Form des aus Rotalgen zu gewinnenden Agar-Agar auch zusammen mit bis zu 30 Gew.-% nicht-gelbildenden Agaropektinen vorliegen können. Hauptbestandteil der Agarosen sind lineare Polysaccharide aus D-Galaktose und 3,6-Anhydro-L-galaktose, die alternierend β-1,3- und β-1,4-glykosidisch verknüpft sind. Die Heteropolysaccharide besitzen vorzugsweise ein Molekulargewicht im Bereich von 110.000 bis 160.000 und sind sowohl farb- als auch geschmacklos. Als Alternativen kommen Pektine, Xanthane (auch Xanthan Gum) sowie deren Mischungen in Frage. Es sind weiterhin solche Typen bevorzugt, die noch in 1-Gew.-%iger wässriger Lösung Gele bilden, die nicht unterhalb von 80 °C schmelzen und sich bereits oberhalb von 40 °C wieder verfestigen. Aus der Gruppe der thermogelierenden Proteine seien exemplarisch die verschiedenen Gelatine-Typen genannt.

***Kationpolymere***

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol. Vorzugsweise wird als Verkapselungsmaterial Chitosan eingesetzt. Chitosane stellen Biopolymere dar und werden zur Gruppe der Hydrokolloide gezählt. Chemisch betrachtet handelt es sich um partiell deacetylierte Chitine unterschiedlichen Molekulargewichtes, die den folgenden - idealisierten - Monomerbaustein enthalten:

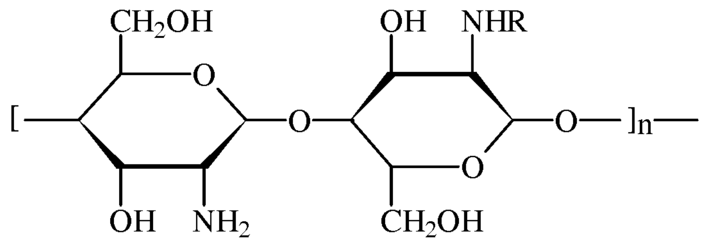

Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt. Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren, das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Vorzugsweise werden solche Typen eingesetzt, wie die ein durchschnittliches Molekulargewicht von 10.000 bis 500.000 bzw. 800.000 bis 1.200.000 Dalton aufweisen und/oder eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% besitzen. Aus Gründen der besseren Wasserlöslichkeit werden die Chitosane in der Regel in Form ihrer Salze, vorzugsweise als Glycolate eingesetzt.

***Anionpolymere***

Die anionischen Polymere haben die Aufgabe, mit den kationischen Membranen zu bilden. Für diesen Zweck eignen sich vorzugsweise Salze der Alginsäure. Bei der Alginsäure handelt es sich um ein Gemisch carboxylgruppenhaltiger Polysaccharide mit folgendem idealisierten Monomerbaustein:

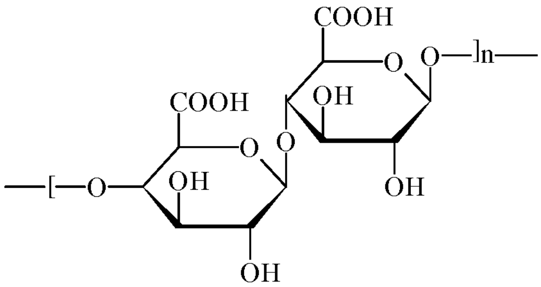

Das durchschnittliche Molekulargewicht der Alginsäuren bzw. der Alginate liegt im Bereich von 150.000 bis 250.000. Dabei sind als Salze der Alginsäure sowohl deren vollständige als auch deren partiellen Neutralisationsprodukte zu verstehen, insbesondere die Alkalisalze und hierunter vorzugsweise das Natriumalginat ("Algin") sowie die Ammonium- und Erdalkalisalze. besonders bevorzugt sind Mischalginate, wie z.B. Natrium/Magnesium- oder Natrium/Calciumalginate. In einer alternativen Ausführungsform der Erfindung kommen für diesen Zweck jedoch auch anionische Chitosanderivate, wie z.B. Carboxylierungs- und vor allem Succinylierungsprodukte in Frage. Alternativ kommen auch Poly(meth)acrylate mit durchschnittlichen Molekulargewichten im Bereich von 5.000 bis 50.000 Dalton sowie die verschiedenen Carboxymethylcellulosen in Frage. Anstelle der anionischen Polymeren können für die Ausbildung der Hüllmembran auch anionische Tenside oder niedermolekulare anorganische Salze, wie beispielsweise Pyrophosphate eingesetzt werden.

***Verkapselung***

Zur Herstellung der Mikrokapseln stellt man üblicherweise eine 1 bis 10, vorzugsweise 2 bis 5 Gew.-%ige wässrige Lösung des Gelbildners, vorzugsweise des Agar-Agars her und erhitzt diese unter Rückfluss. In der Siedehitze, vorzugsweise bei 80 bis 100 °C, wird eine zweite wässrige Lösung zugegeben, welche das Kationpolymer, vorzugsweise das Chitosan in Mengen von 0,1 bis 2, vorzugsweise 0,25 bis 0,5 Gew.-% und den Wirkstoffen in Mengen von 0,1 bis 25 und insbesondere 0,25 bis 10 Gew.-% enthält; diese Mischung wird als Matrix bezeichnet. Die Beladung der Mikrokapseln mit Wirkstoffen kann daher ebenfalls 0,1 bis 25 Gew.-% bezogen auf das Kapselgewicht betragen. Falls gewünscht, können zu diesem Zeitpunkt zur Viskositätseinstellung auch wasserunlösliche Bestandteile, beispielsweise anorganische Pigmente zugegeben werden, wobei man diese in der Regel in Form von wässrigen oder wässrig/alkoholischen Dispersionen zusetzt. Zur Emulgierung bzw. Dispergierung der Wirkstoffe kann es ferner von Nutzen sein, der Matrix Emulgatoren und/oder Lösungsvermittler hinzuzugeben. Nach der Herstellung der Matrix aus Gelbildner, Kationpolymer und Wirkstoffen kann die Matrix optional in einer Ölphase unter starker Scherung sehr fein dispergiert werden, um bei der nachfolgenden Verkapselung möglichst kleine Teilchen herzustellen. Dabei hat es sich als besonders vorteilhaft erwiesen, die Matrix auf Temperaturen im Bereich von 40 bis 60 °C zu erwärmen, während man die Ölphase auf 10 bis 20 °C kühlt. Im letzten, nun wieder obligatorischen Schritt erfolgt dann die eigentliche Verkapselung, d.h. die Ausbildung der Hüllmembran durch Inkontaktbringen des Kationpolymers in der Matrix mit den anionischen Polymeren. Hierzu empfiehlt es sich, die gegebenenfalls in der Ölphase dispergierte Matrix bei einer Temperatur im Bereich von 40 bis 100, vorzugsweise 50 bis 60 °C mit einer wässrigen, etwa 1 bis 50 und vorzugsweise 10 bis 15 Gew.-%ige wässrigen Lösung des Anionpolymers zu behandeln und dabei - falls erforderlich - gleichzeitig oder nachträglich die Ölphase zu entfernen. Die dabei resultierenden wässrigen Zubereitungen weisen in der Regel einen Mikrokapselgehalt im Bereich von 1 bis 10 Gew.-% auf. In manchen Fällen kann es dabei von Vorteil sein, wenn die Lösung der Polymeren weitere Inhaltsstoffe, beispielsweise Emulgatoren oder Konservierungsmittel enthält. Nach Filtration werden Mikrokapseln erhalten, welche im Mittel einen Durchmesser im Bereich von vorzugsweise etwa 0,01 bis 1 mm aufweisen. Es empfiehlt sich, die Kapseln zu sieben, um eine möglichst gleichmäßige Größenverteilung sicherzustellen. Die so erhaltenen Mikrokapseln können im herstellungsbedingten Rahmen eine beliebige Form aufweisen, sie sind jedoch bevorzugt näherungsweise kugelförmig. Alternativ kann man die Anionpolymere auch zur Herstellung der Matrix einsetzen und die Verkapselung mit den Kationpolymeren, speziell den Chitosanen durchführen.

Alternativ kann die Verkapselung auch unter ausschließlicher Verwendung von Kationpolymeren erfolgen, wobei man sich deren Eigenschaft zu Nutze macht, bei pH-Werten oberhalb des pKs-Wertes zu koagulieren.

In einem zweiten alternativen Verfahren wird zur Herstellung der erfindungsgemäßen Mikrokapseln wird zunächst eine O/W-Emulsion zubereitet, welche neben dem Ölkörper, Wasser und den Wirkstoffen eine wirksame Menge Emulgator enthält. Zur Herstellung der Matrix wird diese Zubereitung unter starkem Rühren mit einer entsprechenden Menge einer wässrigen Anionpolymerlösung versetzt. Die Membranbildung erfolgt durch Zugabe der Chitosanlösung. Der gesamte Vorgang findet vorzugsweise im schwach sauren Bereich bei pH = 3 bis 4 statt. Falls erforderlich erfolgt die pH-Einstellung durch Zugabe von Mineralsäure. Nach der Membranbildung wird der pH-Wert auf 5 bis 6 angehoben, beispielsweise durch Zugabe von Triethanolamin oder einer anderen Base. Hierbei kommt es zu einem Anstieg der Viskosität, die durch Zugabe von weiteren Verdickungsmitteln, wie z.B. Polysacchariden, insbesondere Xanthan-Gum, Guar-Guar, Agar-Agar, Alginaten und Tylosen, Carboxymethylcellulose und Hydroxyethylcellulose, höhermolekularen Polyethylenglycolmono- und - diestern von Fettsäuren, Polyacrylaten, Polyacrylamiden und dergleichen noch unterstützt werden kann. Abschließend werden die Mikrokapseln von der wässrigen Phase beispielsweise durch Dekantieren, Filtrieren oder Zentrifugieren abgetrennt.

In einem dritten alternativen Verfahren erfolgt die Bildung der Mikrokapseln um einen vorzugsweise festen, beispielsweise kristallinen Kern, indem dieser schichtweise mit entgegengesetzt geladenen Polyelektrolyten eingehüllt wird. In diesem Zusammenhang sei auf das Europäische Patent EP 1064088 B1 (Max-Planck Gesellschaft) verwiesen.

**Verwendung der Zubereitungen**

Schließlich betrifft die Erfindung weiterhin die Verwendung von Mischungen enthaltend

(a) Menthofuran und

(b) Mentholverbindungen der Formeln (II) und/oder (III)

wobei die Komponenten (a) und (b) im Gewichtsverhältnis 25:75 bis 75:25 vorhanden sind, zur Herstellung von kosmetischen Zubereitungen, pharmazeutischen Zubereitungen sowie Nahrungsmittelzubereitungen.

**Beispiele**

**Beispiele 1 bis 7, Vergleichsbeispiele V1 bis V4**

Kaugummimassen bestehend aus 20 Gew.-% Polyisobutylen (MW 60.000), 51 Gew.-% Sorbitol, 5 Gew.-% Mannitol, 8 Gew.-% Glycerin, 8,2 Gew.-% einer 1:1-Mischung aus Lycasin und Glycerin, 0,2 Gew.-% Lecithin (ad 99,5 Gew.-% Wasser) wurden hergestellt und mit jeweils 0,5 Gew.-% verschiedener synthetischer Aromazubereitungen versetzt. Anschließend wurden die Kaugummimassen von einem Panel bestehend aus 5 geschulten Personen sensorisch auf einer Skala von 1 (kaum zu bemerken) bis 10 (dominant) bewertet. Die Zusammensetzung der Aromakomponenten sowie die Bewertung der einzelnen Geschmacks- und Geruchsnoten (angegeben ist jeweils der Mittelwert der Beurteilungen) sind in Tabelle 1 zusammengefasst. Das Beispiel 2 ist erfindungsgemäß, die Beispiele V1 bis V4 dienen zum Vergleich.

**Tabelle 1**

| Sensorische Bewertung von Kaugummis in Abhängigkeit der Aromakomponente | | | | | |
|---|---|---|---|---|---|
| **Zusammensetzung Aromakomponente** | **V1** | **2** | **V2** | **V3** | **V4** |
| Menthol | 60 | | | | |
| Menthon | 25 | | | | |
| Methylacetat | 5 | | | | |
| Neomenthol | 4 | 3 | 3 | 3 | 3 |
| Isomenthol | 4 | 3 | 3 | 3 | 3 |
| Menthofuran | 2 | 2 | 2 | 2 | 2 |
| Menthone Glyceryl Acetal | - | 2 | - | - | - |
| WS-1 | - | - | 2 | - | - |
| Isopulegol | - | - | - | 2 | - |

(fortgesetzt)

| Sensorische Bewertung von Kaugummis in Abhängigkeit der Aromakomponente | | | | | |
|---|---|---|---|---|---|
| **Zusammensetzung Aromakomponente** | **V1** | **2** | **V2** | **V3** | **V4** |
| Thymol | - | - | - | - | 2 |
| Wasser | Ad 100 | | | | |

**Tabelle 1**

| Sensorische Bewertung von Kaugummis in Abhängigkeit der Aromakomponente (Forts.) | | | | | |
|---|---|---|---|---|---|
| **Sensorische Bewertung** | **V1** | **2** | **V2** | **V3** | **V4** |
| Süß | 4 | 6 | 4 | 4 | 4 |
| Heuartig-minzig | 4 | 8 | 5 | 5 | 5 |
| Scharf | 8 | 4 | 7 | 8 | 8 |
| Stechend | 8 | 4 | 7 | 8 | 8 |
| Bitter | 8 | 4 | 7 | 8 | 8 |
| Teerartig | 7 | 1 | 6 | 6 | 6 |

Die **Kontroll-Formulierung V1** enthielt eine Mischung von Mentholverbindungen wie sie einem klassischen Pfefferminzöl vor der Vakuumdestillation entspricht. Der Gehalt von 2 Gew.-% Menthofuran bewirkte, dass der süße und minzige Geruch des Öls kaum zum Tragen kam, sondern durch scharfe, stechende Noten überlagert wurde; außerdem schmeckte das Produkt bitter und deutlich nach Teer. In dem **erfindungsgemäßen Beispiel 2** wurde ein Teil des Neomenthols und Isomenthols Menthone Glyceryl Acetal ausgetauscht, während der Anteil an Menthofuran unverändert blieb. In der sensorischen Beurteilung hat diese Formulierung deutlich besser abgeschnitten, insbesondere schmeckte die Formulierung eher süß als bitter, der Teergeschmack wurde fast völlig überdeckt und der unangenehme scharfstechende Geruch war deutlich vermindert. In gleicher Weise wurden in den **Vergleichsversuchen V2 bis V4** verschiedene andere Mentholverbindungen eingesetzt. Obschon diese eine geringfügige Verbesserung der sensorischen Eigenschaften bewirkten, waren die Ergebnisse letztlich doch unbefriedigend, d.h. die Kaugummizubereitungen wurden abschließend negativ und nicht für die Vermarktung geeignet bewertet.

**Beispiele 8 bis 14, Vergleichsbeispiele V5 bis V8**

Verschiedene klare O/W-Sonnenschutzemulsionen wurden nach der PIT-Methode durch Vermischen der Komponenten gemäß Tabelle 2 hergestellt:

**Tabelle 2**

| Zusammensetzung O/W-Sonnenschutzlotionen | | |
|---|---|---|
| **Komponente** | **Handelsprodukt** | **Menge [Gew.-%]** |
| Polyglyceryl-2-Polyhydroxystearate (and) Lauryl Glucosides (and) Glycerin | Eumulgin® VL 75 | 2,5 |
| Glyceryl Stearate | Cutina® GMS | 2,0 |
| Cetearyl Alcohol | Lanette® O | 4,0 |
| PVP/Hexadecen Copolymer | Antaron® V216 | 3,0 |
| Cocoglycerides | Myritol® 818 | 6,0 |
| Oleyl Erucate | Cetiol® J600 | 3,0 |

**Tabelle 2**

| Zusammensetzung O/W-Sonnenschutzlotionen (Forts.) | | |
|---|---|---|
| **Komponente** | **Handelsprodukt** | **Menge [Gew.-%]** |
| Dicaprylyl Ether | Cetiol® OE | 5,0 |
| Mineralöl | | 2,0 |
| Bisabolol | | 1,2 |
| Tocopherol | Copherol® F 1300 | 1,0 |
| Octocrylene | Neo Heliopan® 303 | 4,0 |
| Isoamyl-p-methoxycinnamat | Neo Heliopan® E 1000 | 2,0 |
| Octyl-methoycinnamate | Neo Heliopan® AV | 3,0 |
| Octyl Triazon | Uvinul® T15 | 1,0 |
| Aromamischung | | 0,5 |
| Glycerin | | 5,0 |
| Wasser | | Ad 100 |

Die Sonnenschutzlotionen unterschieden sich lediglich im Hinblick auf die Zusammensetzung der Aromamischung, bei der zum einen ein herkömmliches Pfefferminzöl nachgestellt wurde und zum anderen Teile an Neomenthol und Isomenthol gegen verschiedene synthetische Mentholverbindungen ausgetauscht wurden.

Nach der Herstellung wurden die Lotionen in klare PET-Flaschen abgefüllt und diese bei 30 °C gelagert. Anschließend wurden die Lotionen nach 12, 24 und 48 h hinsichtlich ihres Erscheinungsbildes beurteilt. Dabei bedeutet (+) = unverändert; (#) geringfügige Tröpfchenbildung und (-) Abscheidung von Öltröpfchen an der Oberfläche sowie leicht gelbliche Verfärbung. Die Ergebnisse sind in Tabelle 3 zusammengefast. Das Beispiel 9 isterfindungsgemäß, die Beispiele V5 bis V8 dienen wieder zum Vergleich.

**Tabelle 3**

| Bewertung der Lagerstabilität von O/W-Sonnenschutzlotionen in Abhängigkeit der Aromakomponenten | | | | | |
|---|---|---|---|---|---|
| **Zusammensetzung Aromakomponente** | **V5** | **9** | **V6** | **V7** | **V8** |
| Menthol | 60 | | | | |
| Menthon | 25 | | | | |
| Methylacetat | 5 | | | | |
| Neomenthol | 4 | 3 | 3 | 3 | 3 |
| Isomenthol | 4 | 3 | 3 | 3 | 3 |
| Menthofuran | 2 | 2 | 2 | 2 | 2 |
| Menthone Glyceryl Acetal | - | 2 | - | - | - |

**Tabelle 3**

| Bewertung der Lagerstabilität von O/W-Sonnenschutzlotionen in Abhängigkeit der Aromakomponenten | | | | | |
|---|---|---|---|---|---|
| **Zusammensetzung Aromakomponente** | **V5** | **9** | **V6** | **V7** | **V8** |
| WS-1 | - | - | 2 | - | - |
| Isopulegol | - | - | - | 2 | - |
| Thymol | - | - | - | - | 2 |
| Wasser | Ad 100 | | | | |

(fortgesetzt)

| Bewertung | | | | | |
|---|---|---|---|---|---|
| Nach 12 h | # | + | + | + | + |
| Nach 24 h | # | + | # | # | # |
| Nach 48 h | - | + | - | - | - |

Die **Kontroll-Formulierung V5** enthielt wiederum eine Mischung von Mentholverbindungen wie sie einem klassischen Pfefferminzöl vor der Vakuumdestillation entspricht. Der Gehalt von 2 Gew.-% Menthofuran bewirkte, dass bei dem Produkt sehr rasch Öltröpfchen abgeschieden wurden und der Inhalt sich auch leicht ins Gelbliche verfärbte. In dem **erfindungsgemäßen Beispiel 9** wurde wieder ein Teil des Neomenthols und Isomenthols gegen Menthone Glyceryl Acetal ausgetauscht, während der Anteil an Menthofuran unverändert blieb. In der optischen Beurteilung hat diese Formulierung deutlich besser abgeschnitten: die Formulierung erwies sich als vollständig lagerstabil. In gleicher Weise wurden in den **Vergleichsversuchen V5 bis V8** verschiedene andere Mentholverbindungen eingesetzt. Obschon diese eine geringfügige Verbesserung der Lagerstabilität bewirkten, waren die Ergebnisse letztlich doch ähnlich schlecht wie im Fall der Pfefferminzöl-Nachstellung.

In den nachfolgenden Tabellen finden sich zahlreiche Formulierungsbeispiele für kosmetische, pharmazeutische und Nahrungsmittelzubereitungen.

**Tabelle 4**

| Beispiele für kosmetische Zubereitungen (Wasser, Konservierungsmittel ad 100 Gew.-%) | | | | | | | | | | |
|---|---|---|---|---|---|---|---|---|---|---|
| **Zusammensetzung (INCI)** | **1** | **2** | **3** | **4** | **5** | **6** | **7** | **8** | **9** | **10** |
| **Texapon® NSO**<br>Sodium Laureth Sulfate | - | - | - | - | - | - | 38,0 | 38,0 | 25,0 | - |
| **Texapon® SB 3**<br>Disodium Laureth Sulfosuccinate | - | - | - | - | - | - | - | - | 10,0 | - |
| **Plantacare® 818**<br>Coco Glucosides | - | - | - | - | - | - | 7,0 | 7,0 | 6,0 | - |
| **Plantacare® PS 10**<br>Sodium Laureth Sulfate (and) Coco Glucosides | - | - | - | - | - | - | - | - | - | 16,0 |
| **Dehyton® PK 45**<br>Cocamidopropyl Betaine | - | - | - | - | - | - | - | - | 10,0 | - |
| **Dehyquart® A**<br>Cetrimonium Chloride | 2,0 | 2,0 | 2,0 | 2,0 | 4,0 | 4,0 | - | - | - | - |
| **Dehyquart L® 80**<br>Dicocoylmethylethoxymonium Methosulfate (and) Propylenglycol | 1,2 | 1,2 | 1,2 | 1,2 | 0,6 | 0,6 | - | - | - | - |
| **Eumulgin® B2**<br>Ceteareth-20 | 0,8 | 0,8 | - | 0,8 | - | 1,0 | - | - | - | - |
| **Eumulgin® VL 75**<br>Lauryl Glucoside (and) Polyglyceryl-2 Polyhydroxystearate (and) Glycerin | - | - | 0,8 | - | 0.8 | - | - | - | - | - |
| **Lanette® O**<br>Cetearyl Alcohol | 2,5 | 2,5 | 2,5 | 2,5 | 3,0 | 2,5 | - | - | - | - |
| **Cutina® GMS**<br>Glyceryl Stearate | 0,5 | 0,5 | 0,5 | 0,5 | 0,5 | 1,0 | - | - | - | - |
| **Cetiol® HE** | 1,0 | - | - | - | - | - | - | - | 1,0 | |

(fortgesetzt)

| Beispiele für kosmetische Zubereitungen (Wasser, Konservierungsmittel ad 100 Gew.-%) | | | | | | | | | | |
|---|---|---|---|---|---|---|---|---|---|---|
| **Zusammensetzung (INCI)** | **1** | **2** | **3** | **4** | **5** | **6** | **7** | **8** | **9** | **10** |
| PEG-7 Glyceryl Cocoate | | | | | | | | | | |
| **Cetiol® PGL** Hexyldecanol (and) Hexyldecyl Laurate | - | 1,0 | - | - | 1,0 | - | - | - | - | - |
| **Cetiol® V** Decyl Oleate | - | - | - | 1,0 | - | - | - | - | - | - |
| **Eutanol® G** Octyldodecanol | - | - | 1,0 | - | - | 1,0 | - | - | - | - |
| **Nutrilan® Keratin W** Hydrolyzed Keratin | - | - | - | 2,0 | - | - | - | - | - | - |
| **Lamesoft® LMG** Glyceryl Laurate (and) Potassium Cocoyl Hydrolyzed Collagen | - | - | - | - | - | - | 3,0 | 2,0 | 4,0 | - |
| **Euperlan® PK 3000 AM** Glycol Distearate (and) Laureth-4 (and) Cocamidopropyl Betaine | - | - | - | - | - | - | - | 3,0 | 5,0 | 5,0 |
| **Generol® 122 N** Soja Sterol | - | - | - | - | 1,0 | 1,0 | - | - | - | - |
| **Menthofuran/Frescolat MGA (1:1)** | 0,3 | 0,3 | 0,3 | 0,3 | 0,3 | 0,3 | 0,3 | 0,3 | 0,3 | 0,3 |
| **Hydagen® CMF** Chitosan | 1,0 | 1,0 | 1,0 | 1,0 | 1,0 | 1,0 | 1,0 | 1,0 | 1,0 | 1,0 |
| **Copherol® 1250** Tocopherol Acetate | - | - | 0,1 | 0,1 | - | - | - | - | - | - |
| **Arlypon® F** Laureth-2 | - | - | - | - | - | - | 3,0 | 3,0 | 1,0 | - |
| **Sodium Chloride** | - | - | - | - | - | - | - | 1,5 | - | 1,5 |
| (1-4) Haarspülung, (5-6) Haarkur, (7-8) Duschbad, (9) Duschgel, (10) Waschlotion | | | | | | | | | | |

**Tabelle 4**

| Beispiele für kosmetische Zubereitungen (Wasser, Konservierungsmittel ad 100 Gew.-%) (Forts.) | | | | | | | | | | |
|---|---|---|---|---|---|---|---|---|---|---|
| **Zusammensetzung (INCI)** | **11** | **12** | **13** | **14** | **15** | **16** | **17** | **18** | **19** | **20** |
| **Texapon® NSO** Sodium Laureth Sulfate | 20,0 | 20,0 | 12,4 | - | 25,0 | 11,0 | - | - | - | - |
| **Texapon® K 14 S** Sodium Myreth Sulfate | - | - | - | - | - | - | - | - | 11,0 | 23,0 |
| **Texapon® SB 3** Disodium Laureth Sulfosuccinate | - | - | - | - | - | 7,0 | - | - | - | - |
| **Plantacare® 818** Coco Glucosides | 5,0 | 5,0 | 4,0 | - | - | - | - | - | 6,0 | 4,0 |
| **Plantacare® 2000** Decyl Glucoside | - | - | - | - | 5,0 | 4,0 | - | - | - | - |
| **Plantacare® PS 10** | - | - | - | 40,0 | - | - | 16,0 | 17,0 | - | - |

(fortgesetzt)

| Beispiele für kosmetische Zubereitungen (Wasser, Konservierungsmittel ad 100 Gew.-%) (Forts.) | | | | | | | | | | |
|---|---|---|---|---|---|---|---|---|---|---|
| **Zusammensetzung (INCI)** | **11** | **12** | **13** | **14** | **15** | **16** | **17** | **18** | **19** | **20** |
| Sodium Laureth Sulfate (and) Coco Glucosides | | | | | | | | | | |
| **Dehyton® PK 45** Cocamidopropyl Betaine | 20,0 | 20,0 | - | - | 8,0 | - | - | - | - | 7,0 |
| **Eumulgin® B1** Ceteareth-12 | - | - | - | - | 1,0 | - | - | - | - | - |
| **Eumulgin® B2** Ceteareth-20 | - | - | - | 1,0 | - | - | - | - | - | - |
| **Lameform® TGI** Polyglyceryl-3 Isostearate | - | - | - | 4,0 | - | - | - | - | - | - |
| **Dehymuls® PGPH** Polyglyceryl-2 Dipolyhydroxystearate | - | - | 1,0 | - | - | - | - | - | - | - |
| **Monomuls® 90-L 12** Glyceryl Laurate | - | - | - | - | - | - | - | - | 1,0 | 1,0 |
| **Cetiol® HE** PEG-7 Glyceryl Cocoate | - | 0,2 | - | - | - | - | - | - | - | - |
| **Eutanol® G** Octyldodecanol | - | - | - | 3,0 | - | - | - | - | - | - |
| **Nutrilan® Keratin W** Hydrolyzed Keratin | - | - | - | - | - | - | - | - | 2,0 | 2,0 |
| **Nutrilan® I** Hydrolyzed Collagen | 1,0 | - | - | - | - | 2,0 | - | 2,0 | - | - |
| **Lamesoft® LMG** Glyceryl Laurate (and) Potassium Cocoyl Hydrolyzed Collagen | - | - | - | - | - | - | - | - | 1,0 | - |
| **Lamesoft® 156** Hydrogenated Tallow Gyceride (and) Potassium Cocoyl Hydrolyzed Collagen | - | - | - | - | - | - | - | - | - | 5,0 |
| **Gluadin® WK** Sodium Cocoyl Hydrolyzed Wheat Protein | 1,0 | 1,5 | 4,0 | 1,0 | 3,0 | 1,0 | 2,0 | 2,0 | 2,0 | - |
| **Euperlan® PK 3000 AM** Glycol Distearate (and) Laureth-4 (and) Cocamidopropyl Betaine | 5,0 | 3,0 | 4,0 | - | - | - | - | 3,0 | 3,0 | - |
| **Arlypon® F** Laureth-2 | 2,6 | 1,6 | - | 1,0 | 1,5 | - | - | - | - | - |
| **Menthofuran/Frescolat MGA (1:1)** | 0,3 | 0,3 | 0,3 | 0,3 | 0,3 | 0,3 | 0,3 | 0,3 | 0,3 | 0,3 |
| **Hydagen® CMF** Chitosan | 1,0 | 1,0 | 1,0 | 1,0 | 1,0 | 1,0 | 1,0 | 1,0 | 1,0 | 1,0 |
| **Sodium Chloride** | - | - | - | - | - | 1,6 | 2,0 | 2,2 | - | 3,0 |

(fortgesetzt)

| Beispiele für kosmetische Zubereitungen (Wasser, Konservierungsmittel ad 100 Gew.-%) (Forts.) | | | | | | | | | | |
|---|---|---|---|---|---|---|---|---|---|---|
| **Zusammensetzung (INCI)** | **11** | **12** | **13** | **14** | **15** | **16** | **17** | **18** | **19** | **20** |
| **Glycerin** (86 Gew.-%ig) | - | 5,0 | - | - | - | - | - | 1,0 | 3,0 | - |
| (11-14) Duschbad "Two-in-One), (15-20) Shampoo | | | | | | | | | | |

**Tabelle 4**

| Beispiele für kosmetische Zubereitungen (Wasser, Konservierungsmittel ad 100 Gew.-%) (Forts.) | | | | | | | | | | |
|---|---|---|---|---|---|---|---|---|---|---|
| **Zusammensetzung (INCI)** | **21** | **22** | **23** | **24** | **25** | **26** | **27** | **28** | **29** | **30** |
| **Texapon® NSO** Sodium Laureth Sulfate | - | 30,0 | 30,0 | - | 25,0 | - | - | - | - | - |
| **Plantacare® 818** Coco Glucosides | - | 10,0 | - | - | 20,0 | - | - | - | - | - |
| **Plantacare® PS 10** Sodium Laureth Sulfate (and) Coco Glucosides | 22,0 | - | 5,0 | 22,0 | - | - | - | - | - | - |
| **Dehyton® PK 45** Cocamidopropyl Betaine | 15,0 | 10,0 | 15,0 | 15,0 | 20,0 | - | - | - | - | - |
| **Emulgade® SE** Glyceryl Sterate (and) Ceteareth 12/20 (and) Cetearyl Alcohol (and) Cetyl Palmitate | - | - | - | - | - | 5,0 | 5,0 | 4,0 | - | - |
| **Eumulgin® B1** Ceteareth-12 | - | - | - | - | - | - | - | 1,0 | - | - |
| **Lameform® TGI** Polyglyceryl-3 Isostearate | - | - | - | - | - | - | - | - | 4,0 | - |
| **Dehymuls® PGPH** Polyglyceryl-2 Dipolyhydroxystearate | - | - | - | - | - | - | - | - | - | 4,0 |
| **Monomuls® 90-O 18** Glyceryl Oleate | - | - | - | - | - | - | - | - | 2,0 | - |
| **Cetiol® HE** PEG-7 Glyceryl Cocoate | 2,0 | - | - | 2,0 | 5,0 | - | - | - | - | 2,0 |
| **Cetiol® OE** Dicaprylyl Ether | - | - | - | - | - | - | - | - | 5,0 | 6,0 |
| **Cetiol® PGL** Hexyldecanol (and) Hexyldecyl Laurate | - | - | - | - | - | - | - | 3,0 | 10,0 | 9,0 |
| **Cetiol® SN** Cetearyl Isononanoate | - | - | - | - | - | 3,0 | 3,0 | - | - | - |
| **Cetiol® V** Decyl Oleate | - | - | - | - | - | 3,0 | 3,0 | - | - | - |
| **Myritol® 318** Coco Caprylate Caprate | - | - | - | - | - | - | - | 3,0 | 5,0 | 5,0 |
| **Bees Wax** | - | - | - | - | - | - | - | - | 7,0 | 5,0 |

(fortgesetzt)

| Beispiele für kosmetische Zubereitungen (Wasser, Konservierungsmittel ad 100 Gew.-%) (Forts.) | | | | | | | | | | |
|---|---|---|---|---|---|---|---|---|---|---|
| **Zusammensetzung (INCI)** | **21** | **22** | **23** | **24** | **25** | **26** | **27** | **28** | **29** | **30** |
| **Nutrilan® Elastin E20** Hydrolyzed Elastin | - | - | - | - | - | 2,0 | - | - | - | - |
| **Nutrilan® I-50** Hydrolyzed Collagen | - | - | - | - | 2,0 | - | 2,0 | - | - | - |
| **Gluadin® AGP** Hydrolyzed Wheat Gluten | 0,5 | 0,5 | 0,5 | - | - | - | - | 0,5 | - | - |
| **Gluadin® WK** Sodium Cocoyl Hydrolyzed Wheat Protein | 2,0 | 2,0 | 2,0 | 2,0 | 5,0 | - | - | - | 0,5 | 0,5 |
| **Euperlan® PK 3000 AM** Glycol Distearate (and) Laureth-4 (and) Cocamidopropyl Betaine | 5,0 | - | - | 5,0 | - | - | - | - | - | - |
| **Arlypon® F** Laureth-2 | - | - | - | - | - | - | - | - | - | - |
| **Retinol-Nanokapseln gem. Bsp. 3** | 1,0 | 1,0 | 1,0 | 1,0 | 1,0 | 1,0 | 1,0 | 1,0 | 1,0 | 1,0 |
| **Hydagen® CMF** Chitosan | 1,0 | 1,0 | 1,0 | 1,0 | 1,0 | 1,0 | 1,0 | 1,0 | 1,0 | 1,0 |
| **Menthofuran/Frescolat MGA (1:1)** | 0,3 | 0,3 | 0,3 | 0,3 | 0,3 | 0,3 | 0,3 | 0,3 | 0,3 | 0,3 |
| **Glycerin** (86 Gew.-%ig) | - | - | - | - | - | 3,0 | 3,0 | 5,0 | 5,0 | 3,0 |
| (21-25) Schaumbad, (26) Softcreme, (27, 28) Feuchtigkeitsemulsion, (29, 30) Nachtcreme | | | | | | | | | | |

**Tabelle 4**

| Beispiele für kosmetische Zubereitungen (Wasser, Konservierungsmittel ad 100 Gew.-%) (Forts.) | | | | | | | | | | |
|---|---|---|---|---|---|---|---|---|---|---|
| **Zusammensetzung (INCI)** | **31** | **32** | **33** | **34** | **35** | **36** | **37** | **38** | **39** | **40** |
| **Dehymuls® PGPH** Polyglyceryl-2 Dipolyhydroxystearate | 4,0 | 3,0 | - | 5,0 | - | - | - | - | - | - |
| **Lameform® TGI** Polyglyceryl-3 Diisostearate | 2,0 | 1,0 | - | - | - | - | - | - | - | - |
| **Emulgade® PL 68/50** Cetearyl Glucoside (and) Cetearyl Alcohol | - | - | - | - | 4,0 | - | - | - | 3,0 | - |
| **Eumulgin®B2** Ceteareth-20 | - | - | - | - | - | - | - | 2,0 | - | - |
| **Tegocare® PS** Polyglyceryl-3 Methylglucose Distearate | - | - | 3,0 | - | - | - | 4,0 | - | - | - |
| **Eumulgin VL 75** Polyglyceryl-2 Dipolyhydroxystearate (and) Lauryl Glucoside (and) Glycerin | - | - | - | - | - | 3,5 | - | - | 2,5 | - |
| **Bees Wax** | 3,0 | 2,0 | 5,0 | 2,0 | - | - | - | - | - | - |
| **Cutina® GMS** Glyceryl Stearate | - | - | - | - | - | 2,0 | 4,0 | - | - | 4,0 |

(fortgesetzt)

| Beispiele für kosmetische Zubereitungen (Wasser, Konservierungsmittel ad 100 Gew.-%) (Forts.) | | | | | | | | | | |
|---|---|---|---|---|---|---|---|---|---|---|
| **Zusammensetzung (INCI)** | **31** | **32** | **33** | **34** | **35** | **36** | **37** | **38** | **39** | **40** |
| **Lanette® O**<br>Cetearyl Alcohol | - | - | 2,0 | - | 2,0 | 4,0 | 2,0 | 4,0 | 4,0 | 1,0 |
| **Antaron® V 216**<br>PVP / Hexadecene Copolymer | - | - | - | - | - | 3,0 | - | - | - | 2,0 |
| **Myritol® 818**<br>Cocoglycerides | 5,0 | - | 10,0 | - | 8,0 | 6,0 | 6,0 | - | 5,0 | 5,0 |
| **Finsolv® TN**<br>C12/15 Alkyl Benzoate | - | 6,0 | - | 2,0 | - | - | 3,0 | - | - | 2,0 |
| **Cetiol® J 600**<br>Oleyl Erucate | 7,0 | 4,0 | 3,0 | 5,0 | 4,0 | 3,0 | 3,0 | - | 5,0 | 4,0 |
| **Cetiol® OE**<br>Dicaprylyl Ether | 3,0 | - | 6,0 | 8,0 | 6,0 | 5,0 | 4,0 | 3,0 | 4,0 | 6,0 |
| **Mineral Oil** | - | 4,0 | - | 4,0 | - | 2,0 | - | 1,0 | - | - |
| **Cetiol® PGL**<br>Hexadecanol (and) Hexyldecyl Laurate | - | 7,0 | 3,0 | 7,0 | 4,0 | - | - | - | 1,0 | - |
| **Bisabolol** | 1,2 | 1,2 | 1,2 | 1,2 | 1,2 | 1,2 | 1,2 | 1,2 | 1,2 | 1,2 |
| **Menthofuran/Frescolat MGA (1:1)** | 0,3 | 0,3 | 0,3 | 0,3 | 0,3 | 0,3 | 0,3 | 0,3 | 0,3 | 0,3 |
| **Hydagen® CMF**<br>Chitosan | 1,0 | 1,0 | 1,0 | 1,0 | 1,0 | 1,0 | 1,0 | 1,0 | 1,0 | 1,0 |
| **Copherol® F 1300**<br>Tocopherol / Tocopheyl Acetate | 0,5 | 1,0 | 1,0 | 2,0 | 1,0 | 1,0 | 1,0 | 2,0 | 0,5 | 2,0 |
| **Neo Heliopan® Hydro**<br>Sodium Phenylbenzimidazole Sulfonate | 3,0 | - | - | 3,0 | - | - | 2,0 | - | 2,0 | - |
| **Neo Heliopan® 303**<br>Octocrylene | - | 5,0 | - | - | - | 4,0 | 5,0 | - | - | 10,0 |
| **Neo Heliopan® BB**<br>Benzophenone-3 | 1,5 | - | - | 2,0 | 1,5 | - | - | - | 2,0 | - |
| **Neo Heliopan® E 1000**<br>Isoamyl p-Methoxycinnamate | 5,0 | - | 4,0 | - | 2,0 | 2,0 | 4,0 | 10,0 | - | - |
| **Neo Heliopan® AV**<br>Octyl Methoxycinnamate | 4,0 | - | 4,0 | 3,0 | 2,0 | 3,0 | 4,0 | - | 10,0 | 2,0 |
| **Uvinul® T 150**<br>Octyl Triazone | 2,0 | 4,0 | 3,0 | 1,0 | 1,0 | 1,0 | 4,0 | 3,0 | 3,0 | 3,0 |
| **Zinc Oxide** | - | 6,0 | 6,0 | - | 4,0 | - | - | - | - | 5,0 |
| **Titanium Dioxide** | - | - | - | - | - | - | - | 5,0 | - | - |
| **Glycerin (86 Gew.-%ig)** | 5,0 | 5,0 | 5,0 | 5,0 | 5,0 | 5,0 | 5,0 | 5,0 | 5,0 | 5,0 |
| (31) W/O-Sonnenschutzcreme, (32-34) W/O-Sonnenschutzlotion, (35, 38, 40) O/W-Sonnenschutzlotion<br>(36, 37, 39) O/W-Sonnenschutzcreme | | | | | | | | | | |

**Beispiele 15 bis 21, Vergleichsbeispiel V9**

Zur Bestimmung der Reduktion der Apatitlöslichkeit wurde zunächst ein Blindversuch durchgeführt. Hierzu

wurde in einem Reaktionsgefäß mit 300 entsalzten Wasser, thermostatiert auf 37 °C 0,5 g Hydroxylapatitpulver (spez. Oberfläche 60 $m^2$/g, Fa. Merck) suspendiert. Der pH-Wert der Suspension wurde mittels einer automatischen Bürette, mit der Milchsäurelösung zugegeben werden konnte, auf einen konstanten Wert von 5 gehalten. Die zur pH-Stabilisierung verbrauchte Menge 0,1 molarer Milchsäure wurde von einem Schreiber registriert. Der nach 2 Stunden registrierte Verbrauch an Milchsäure entsprach der Löslichkeit des unbehandelten Hydroxylapatitpulvers ($L_u$).

Anschließend wurde der Versuch unter Zugabe von 50 bzw. 150 mg der zu untersuchenden Wirkstoffgemische wiederholt. Der nach zwei Stunden registrierte Verbrauch an Milchsäure entsprach der Löslichkeit des behandelten Hydroxylapatitpulvers ($L_b$). Die Reduktion der Apatitlöslichkeit (ALR in %) durch den Wirkstoff berechnete sich nach:

$$ALR\ (\%) = (L_u - L_b) * 100 / L_u\ (\%)$$

Die Ergebnisse der Messungen sind in Tabelle 5 zusammengefasst.

Zur Bestimmung der Inhibierung des Kristallwachstums (KWI in %) von Hydroxylapatit wurde ebenfalls zunächst ein Blindversuch durchgeführt. Hierzu wurden in einem Reaktionsgefäß 400 ml einer 0,0008 molaren Lösung von $KH_2PO_4$ und 45 ml einer 0,012 molaren Lösung von $CaCl_2$ vorgelegt. Diese Lösung wurde durch Titration mit einer 0,05 molaren Lösung von KOH auf einen pH-Wert von 7,4 eingestellt. Nach Erhalt eines über mindestens 30 Minuten stabilen pH-Wertes wurden 100 mg Hydroxylapatitpulver (spez. Oberfläche 60 $m^2$/g, Fa. Merck) zugegeben. Der pH-Wert der Suspension wurde mittels einer automatischen Bürette, mit der 0,05 molare KOH-Lösung zugegeben werden kann, auf einen konstanten Wert von 7,4 gehalten. Die zur pH-Stabilisierung verbrauchte Menge 0,05 molarer KOH-Lösung wurde von einem Schreiber registriert. Der nach 2 Stunden registrierte Verbrauch an KOH-Lösung ($K_u$) entsprach der Bildung von Hydroxylapatit (Wachstum der Kristalle der Suspension).

Anschließend wurde der Versuch unter Zugabe von 6 bzw. 30 mg des zu untersuchenden Wirkstoffs wiederholt. Der nach 2 Stunden registrierte Verbrauch an 0,05 molarer KOH-Lösung ($K_b$) entsprach der Bildung von Hydroxylapatit (Wachstum der Kristalle in der Suspension) unter dem Einfluss des Wirkstoffs. Die Inhibierung des Kristallwachstums durch den Wirkstoff berechnet sich nach:

$$KWI\ (\%) = (K_u - K_b) * 100 / K_u\ (\%)$$

Die Ergebnisse der Messungen sind in Tabelle 5 zusammengefasst.

**Tabelle 5**

| Apatitlöslichkeit und Kristallwachstumsinhibierung in Abhängigkeit der Aromakomponente | | |
|---|---|---|
| **Zusammensetzung Aromakomponente** | **V9** | **16** |
| Menthol | 60 | |
| Menthon | 25 | |
| Methylacetat | 5 | |
| Neomenthol | 4 | 3 |

**Tabelle 5**

| Apatitlöslichkeit und Kristallwachstumsinhibierung in Abhängigkeit der Aromakomponente (Forts.) | | |
|---|---|---|
| **Zusammensetzung Aromakomponente** | **V9** | **16** |
| Isomenthol | 4 | 3 |
| Menthofuran | 2 | 2 |
| Menthone Glyceryl Acetal | - | 2 |
| Wasser | Ad 100 | |
| **Apatitlöslichkeit [%]** | | |
| 30 mg | 2 | 10 |

(fortgesetzt)

| Apatitlöslichkeit [%] | | |
|---|---|---|
| 150 mg | 12 | 21 |
| **Kristallwachstuminhibierung [%]** | | |
| 5 mg | 3 | 17 |
| 6 mg | 14 | 30 |

Das Beispiel 16 zeigt, dass die erfindungsgemäße Zubereitung gegenüber einer Aromamischung, die herkömmlichem Pfefferminzöl entspricht, über eine deutlich höhere Apatitlöslichkeit und eine stärkere Inhibierung der Bildung von Apatitkristallen verfügt. Mund- und Zahnputzmittel, die derartige Mischung enthalten, zeichnen sich durch eine verbesserte Wirkung gegen Zahnsteinbildung aus.

Die folgende Tabelle 6 enthält eine Reihe von Formulierungsbeispielen für Zahnpasten und Mundwässer.

**Tabelle 6a**

| Zusammensetzung Zahnpaste | | |
|---|---|---|
| **Komponente** | **Handelsprodukt** | **Menge [Gew.-%]** |
| Fällungskieselsäure | Sident® 12 DS | 18,0 |
| Verdickungskieselsäure | Aerosil® 200 | 0,8 |
| Sorbit | | 17,5 |
| Glycerin | | 17,5 |
| Carboxymethylcellulose | Relatin® 100 SR | 0,9 |
| Natriumlaurylsulfat | Texapon® K1296 | 2,0 |
| Natriumfluorid | | 0,22 |
| Saccharin-Natrium | | 0,2 |
| Aromamischung | | 1,0 |
| Wasser | | Ad 100 |

**Tabelle 6b**

| Zusammensetzung Mundwasser | | |
|---|---|---|
| **Komponente** | **Handelsprodukt** | **Menge [Gew.-%]** |
| Ethanol (96%ig) | | 10,0 |
| Sorbitanmonolaurat+20EO | Tween® 20 | 0,4 |
| Aromamischung | | 0,3 |
| Sorbit (70%ige wässrige Lösung) | | 8,0 |
| p-Hydroxybenzoesäuremethylester | | 0,2 |
| Wasser | | Ad 100 |

In der folgenden Tabelle 7 ist schließlich eine Reihe von Beispielformulierungen für Kaugummimassen zusammengestellt.

**Tabelle 7**

| Kaugummimassen | | | | | | | | |
|---|---|---|---|---|---|---|---|---|
| **Zusammensetzung** | **A** | **B** | **C** | **D** | **E** | **F** | **G** | **H** |
| Polyisobutylen (MW 20.000) | 30,0 | 30,0 | 30,0 | 40,0 | 20.0 | 20.0 | 25.0 | 30.0 |
| Glucose | 51,0 | 51,0 | 51,0 | 42,5 | | | | |
| Kornsirup | 10,0 | 10,0 | 10,0 | 8,0 | | | | |
| Sorbitol | | | | | 51,0 | 51,0 | 47,5 | 44,5 |
| Mannitol | | | | | 5,0 | 5,0 | 4,3 | 3,6 |
| Glycerin | 1,8 | 1,8 | 1,8 | 1,8 | 8,0 | 8,0 | 8,0 | 7,0 |
| Lycasin:Glycerin (1:1) | | | | | 8,2 | 8,2 | 8,0 | 7,0 |
| Lecithin | | | | | 0,2 | 0,2 | 0,2 | 0,2 |
| Aromamischung | 1,0 | 1,0 | 1,0 | 1,0 | 1,0 | 1,0 | 1,0 | 1,0 |
| Wasser | Ad 100 | | | | | | | |

**Patentansprüche**

**1.** Zubereitungen, enthaltend

(a) Menthofuran und
(b) Mentholverbindungen der Formeln (II) und/oder (III)

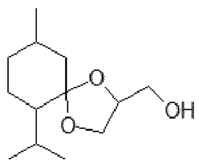

(II)

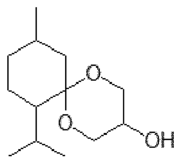

(III)

mit der Maßgabe, dass die Komponenten (a) und (b) im Gewichtsverhältnis 25:75 bis 75:25 vorhanden sind.

**2.** Zubereitungen nach Anspruch 1, **dadurch gekennzeichnet, dass** sie als Komponente (b) Mentholverbindungen enthalten, die ausgewählt sind aus der Gruppe, die gebildet wird von Menthone Glyceryl Acetal (FEMA GRAS 3807), Menthone Glyceryl Ketal (FEMA GRAS 3808) deren Gemischen.

**3.** Zubereitungen nach den Ansprüchen 1 und/oder 2, **dadurch gekennzeichnet, dass** sie einen kosmetischen Zusatzstoff (Komponente c) enthalten, der ausgewählt ist aus der Gruppe, die gebildet wird von Tensiden, Ölkörpern, Emulgatoren, Perlglanzwachsen, Konsistenzgebern, Verdickungsmitteln, Überfettungsmitteln, Stabilisatoren, Polymeren, Siliconverbindungen, Fetten, Wachsen, Lecithinen, Phospholipiden, UV-Lichtschutzfaktoren, Feuchthaltemitteln, biogenen Wirkstoffen, Antioxidantien, Deodorantien, Antitranspirantien, Antischuppenmitteln, Filmbildnern, Quellmitteln, Insektenrepellentien, Selbstbräunern, Tyrosininhibitoren (Depigmentierungsmittel), Hydrotropen, Solubilisatoren, Konservierungsmitteln, Parfümölen und Farbstoffen sowie deren Gemischen.

**4.** Zubereitungen nach mindestens einem der Ansprüche 1 bis 3, **dadurch gekennzeichnet, dass** sie die Komponenten (a) und (b) im Gewichtsverhältnis 40:60 bis 60:40 enthalten.

**5.** Zubereitungen nach mindestens einem der Ansprüche 1 bis 4, **dadurch gekennzeichnet, dass** sie die Komponenten (a+b) und (c) im Gewichtsverhältnis 0.01:99,9 bis 2:98 enthalten.

**6.** Kosmetische Zubereitungen, enthaltend

(a) Menthofuran,
(b) Mentholverbindungen der Formeln (II) und/oder (III) sowie
(c) einen für kosmetische Anwendungen zugelassenen Träger,

mit der Maßgabe, dass die Komponenten (a) und (b) im Gewichtsverhältnis 25:75 bis 75:25 vorhanden sind.

7. Kosmetische Zubereitungen nach Anspruch 6, **dadurch gekennzeichnet, dass** der Träger ausgewählt ist aus der Gruppe, die gebildet wird von Wasser, Alkoholen mit 2 bis 6 Kohlenstoffatomen, Polyolen mit 1 bis 10 Kohlenstoffatomen und 2 bis 4 Hydroxylgruppen sowie Ölkörpern.

8. Kosmetische Zubereitungen nach den Ansprüchen 6 und/oder 7, **dadurch gekennzeichnet, dass** es sich um Produkte handelt, die ausgewählt sind aus der Gruppe, die gebildet wird von Hautpflegemitteln, Haarpflegemitteln, Körperpflegemitteln, Sonnenschutzmitteln sowie Mund- und Zahnpflegemitteln.

9. Pharmazeutische Zusammensetzung, enthaltend

(a) Menthofuran,
b) Mentholverbindungen der Formeln (II) und/oder (III) sowie
(c) einen für pharmazeutische Anwendungen zugelassenen Träger

zur Behandlung von Erkältungszuständen. mit der Maßgabe, dass die Komponenten (a) und (b) im Gewichtsverhältnis 25:75 bis 75:25 vorhanden sind.

10. Pharmazeutische Zusammensetzung, nach Anspruch 9, **dadurch gekennzeichnet, dass** der Träger ausgewählt ist aus der Gruppe, die gebildet wird von Wasser, Alkoholen mit 2 bis 6 Kohlenstoffatomen, Polyolen mit 1 bis 10 Kohlenstoffatomen und 2 bis 4 Hydroxylgruppen sowie Ölkörpern.

11. Pharmazeutische Zusammensetzung, nach den Ansprüchen 9 und/oder 10, **dadurch gekennzeichnet, dass** es sich um Produkte handelt, die ausgewählt sind aus der Gruppe, die gebildet wird von Lutschpastillen, Erkältungsbonbons, Erkältungssäfte, Erkältungssalben und Erkältungssprays.

12. **(Geändert)** Nahrungsmittelzubereitungen, enthaltend

(a) Menthofuran,
(b) Mentholverbindungen der Formeln (II) und/oder (III) sowie
(c) einen für Nahrungsmittelzwecke zugelassenen Träger,

mit der Maßgabe, dass die Komponenten (a) und (b) im Gewichtsverhältnis 25:75 bis 75:25 vorhanden sind.

13. Nahrungsmittelzubereitungen nach Anspruch 12, **dadurch gekennzeichnet, dass** der Träger ausgewählt ist aus der Gruppe, die gebildet wird von Wasser, Ethanol und Glycerin.

14. Nahrungsmittelzubereitungen nach den Ansprüchen 12 und/oder 13, **dadurch gekennzeichnet, dass** es sich um Produkte handelt, die ausgewählt sind aus der Gruppe, die gebildet wird von Getränken, Milchprodukten, Backwaren, Kaugummis und Bonbons.

15. Verwendung von Mischungen enthaltend

(a) Menthofuran und
(b) Mentholverbindungen der Formeln (II) und/oder (III)

wobei die Komponenten (a) und (b) im Gewichtsverhältnis 25:75 bis 75:25 vorhanden sind, zur Herstellung von kosmetischen Zubereitungen, pharmazeutischen Zubereitungen sowie Nahrungsmittelzubereitungen.

**Claims**

1. Compositions comprising

(a) menthofuran, and
(b)menthol compounds corresponding to the formulas (II) and/or (III)

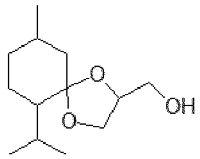

(II)

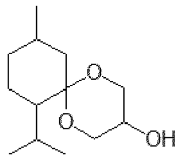

(III)

wherein components (a) and (b) are present in the weight ratio of from 25:75 to 75:25.

**2.** The compositions according to Claim 1, comprising, as component (b) menthol compounds selected from the group consisting of menthol methyl ether, menthone glyceryl acetal (FEMA GRAS 3807), menthone glyceryl ketal (FEMA GRAS 3808) and mixtures thereof.

**3.** The compositions according to Claim 1 and/or 2, additionally comprising a cosmetic additive (component c), which is selected from the group consisting of surfactants, oil bodies, emulsifiers, pearlizing waxes, consistency factors, thickeners, superfatting agents, stabilizers, polymers, silicon compounds, fats, waxes, lecithins, phospholipids, UV protection factors, moisturizers, biogenic agents, antioxidants, deodorants, antiperspirants, anti-dandruff agents, film formers, swelling agents, insect repellants, self-tanning agents, tyrosine inhibitors, (depigmenting agents), hydrotropes, solubilizers, preservatives, perfume oils and dyes and mixtures thereof.

**4.** The compositions according to at least one of claims 1 to 3, wherein components (a) and (b) are present in the weight ratio of from 40:60 to 60:40.

**5.** The compositions according to at least one of claims 1 to 4, wherein components (a+b) and (c) are present in the weight ratio of from 0.01:99.2 to 2:98.

**6.** Cosmetic compositions, comprising

(a) menthofuran,
(b) menthol compounds corresponding to the formulas (II) and/or (III), and
(c) a carrier approved for cosmetic applications.

wherein components (a) and (b) are present in the weight ratio of from 25:75 to 75:25.

**7.** The cosmetic composition according to claim 6, wherein the carrier is selected from the group consisting of water, alcohols containing 2 to 6 carbon atoms, polyols containing 1 to 10 carbon atoms and 2 to 4 hydroxyl groups and oil bodies.

**8.** The cosmetic composition of claim 6, wherein the cosmetic composition is a product selected from the group consisting of skin care products, hair care products, sun screen products and oral and dental care products.

**9.** Pharmaceutical composition, comprising

(a) menthofuran,
(b) menthol compounds corresponding to the formulas (II) and/or (III), and
(c) a carrier approved for pharmaceutical applications.

for treatment of cold symptoms wherein components (a) and (b) are present in the weight ratio of from 25:75 to 75:25.

**10.** Pharmaceutical composition according to claim 9, wherein the carrier is selected from the group consisting of water, alcohols containing 2 to 6 carbon atoms, polyols containing 1 to 10 carbon atoms and 2 to 4 hydroxyl groups and oil bodies.

**11.** Pharmaceutical composition according to claims 9 and/or 10, wherein the pharmaceutical composition is a product

selected from the group consisting of lozenges, cold drops, syrups, cold balms and cold sprays.

**12.** Food compositions, comprising

(a) menthofuran,
(b) menthol compounds corresponding to the formulas (II) and/or (III), and
(c) a carrier approved for food purposes,

wherein components (a) and (b) are present in the weight ratio of from 25:75 to 75:25.

**13.** The food compositions of Claim 12, wherein the carrier is selected from the group consisting of water, ethanol and glycerol.

**14.** The food compositions according to claims 12 and/or 13, wherein the food compositions are products selected from the group consisting of beverages, milk products, bakery products, chewing gums and bonbons.

**15.** Use of compositions, comprising

(a) menthofuran, and
(b) menthol compounds corresponding to the formulas (II) and/or (III),

wherein components (a) and (b) are present in the weight ratio of from 25:75 to 75:25 for manufacture of cosmetic compositions, pharmaceutical compositions and food compositions.

**Revendications**

**1.** Préparations, contenant :

(a) du menthofurane et
(b) des composés de menthol de formule (II) et/ou (III)

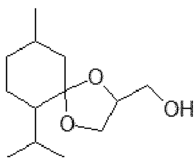
(II)

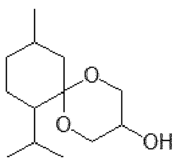
(III)

à condition que les composants (a) et (b) soient présents en un rapport en poids de 25:75 à 75:25.

**2.** Préparations selon la revendication 1, **caractérisées en ce qu'**elles contiennent en tant que composant (b) des composés de menthol qui sont choisis dans le groupe formé par le menthone glycéryl acétal (FEMA GRAS 3807), le menthone glycéryl cétal (FEMA GRAS 3808) et leurs mélanges.

**3.** Préparations selon les revendications 1 et/ou 2, **caractérisées en ce qu'**elles contiennent un additif cosmétique (composant c), qui est choisi dans le groupe formé par les tensioactifs, les corps huileux, les émulsifiants, les cires nacrées, les agents donnant de la consistance, les épaississants, les agents surgraissants, les stabilisateurs, les polymères, les composés de silicone, les graisses, les cires, les lécithines, les phospholipides, les facteurs de photoprotection UV, les agents de rétention de l'humidité, les agents actifs biogènes, les antioxydants, les déodorants, les anti-transpirants, les agents antipelliculaires, les agents filmogènes, les agents gonflants, les agents répulsifs contre les insectes, les autobronzants, les inhibiteurs de tyrosine (agent de dépigmentation), les hydrotropes, les solubilisateurs, les conservateurs, les huiles parfumées et les colorants, ainsi que leurs mélanges.

**4.** Préparations selon au moins l'une quelconque des revendications 1 à 3, **caractérisées en ce qu'**elles contiennent les composants (a) et (b) en un rapport en poids de 40:60 à 60:40.

**5.** Préparations selon au moins l'une quelconque des revendications 1 à 4, **caractérisées en ce qu'**elles contiennent les composants (a+b) et (c) en un rapport en poids de 0,01:99,9 à 2:98.

**6.** Préparations cosmétiques, contenant :

(a) du menthofurane et
(b) des composés de menthol de formule (II) et/ou (III), et
(c) un véhicule admissible pour les applications cosmétiques,

à condition que les composants (a) et (b) soient présents en un rapport en poids de 25:75 à 75:25.

**7.** Préparations cosmétiques selon la revendication 6, **caractérisées en ce que** le véhicule est choisi dans le groupe formé par l'eau, les alcools de 2 à 6 atomes de carbone, les polyols de 1 à 10 atomes de carbone et 2 à 4 groupes hydroxyle, ainsi que les corps huileux.

**8.** Préparations cosmétiques selon les revendications 6 et/ou 7, **caractérisées en ce qu'**il s'agit de produits choisis dans le groupe formé par les agents de soin de la peau, les agents de soin des cheveux, les agents de soin du corps, les agents de protection contre le soleil, ainsi que les agents de soin de la bouche et des dents.

**9.** Composition pharmaceutique, contenant :

(a) du menthofurane et
(b) des composés de menthol de formule (II) et/ou (III), et
(c) un véhicule admissible pour les applications pharmaceutiques,

pour le traitement d'états de refroidissement, à condition que les composants (a) et (b) soient présents en un rapport en poids de 25:75 à 75:25.

**10.** Composition pharmaceutique selon la revendication 9, **caractérisée en ce que** le véhicule est choisi dans le groupe formé par l'eau, les alcools de 2 à 6 atomes de carbone, les polyols de 1 à 10 atomes de carbone et 2 à 4 groupes hydroxyle, ainsi que les corps huileux.

**11.** Composition pharmaceutique selon les revendications 9 et/ou 10, **caractérisée en ce qu'**il s'agit de produits choisis dans le groupe formé par les pastilles perlinguales, les bonbons pour les refroidissements, les sirops pour les refroidissements, les onguents pour les refroidissements et les pulvérisations pour les refroidissements.

**12.** Préparations alimentaires (modifiées), contenant

(a) du menthofurane et
(b) des composés de menthol de formule (II) et/ou (III), et
(c) un véhicule admissible pour les applications alimentaires,

à condition que les composants (a) et (b) soient présents en un rapport en poids de 25:75 à 75:25.

**13.** Préparations alimentaires selon la revendication 12, **caractérisées en ce que** le support est choisi dans le groupe formé par l'eau, l'éthanol et la glycérine.

**14.** Préparations alimentaires selon les revendications 12 et/ou 13, **caractérisées en ce qu'**il s'agit de produits choisis dans le groupe formé par les boissons, les produits laitiers, les produits de boulangerie, les gommes à mâcher et les bonbons.

**15.** Utilisation de mélanges contenant

(a) du menthofurane et
(b) des composés de menthol de formule (II) et/ou (III),

les composants (a) et (b) étant présents en un rapport en poids de 25:75 à 75:25, pour la fabrication de préparations cosmétiques, de préparations pharmaceutiques et de préparations alimentaires.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2006116436 A1
- US 20100273887 A1
- US 20110081303 A1
- US 3488419 A
- DE 2224430 A1
- DE 2343196 A1
- US 5286500 A
- WO 2002091849 A1
- WO 2001001926 A
- WO 2001001927 A
- WO 2001001928 A
- WO 2001001929 A, Cognis
- EP 1064088 B1, Max-Planck Gesellschaft


### In der Beschreibung aufgeführte Nicht-Patentliteratur


- **R.LOCHHEAD.** *Cosm.Toil.,* 1993, vol. 108, 95
- Kosmetische Färbemittel" der Farbstoffkommission der Deutschen Forschungsgemeinschaft. Verlag Chemie, 1984, 81-106